# EUROPEAN PATENT APPLICATION

(11) **EP 2 418 200 A1**
(43) Date of publication of application: **15.02.2012**
(21) Application number: 10761130.3
(22) Date of filing: 07.04.2010
(51) Int. Cl.: C07D 209/48, C07D 209/28, C07C 229/42, C07C 229/40, C07C 57/40, C07C 65/10, C07C 65/34, C07C 209/28, C07C 245/08, A61K 31/4035, A61K 31/196, A61K 31/192, A61K 31/60, A61K 31/405, A61K 31/655, A61K 31/635, A61P 29/00, A61P 7/02

(54) **PHTHALIMIDE DERIVATIVES OF NON-STEROIDAL ANTI-INFLAMMATORY COMPOUNDS AND/OR TNF- MODULATORS, METHOD FOR PRODUCING SAME, PHARMACEUTICAL COMPOSITIONS CONTAINING SAME AND USES THEREOF FOR THE TREATMENT OF INFLAMMATORY DISEASES**

(30) Priority: 06.04.2009 BR PI0901298
(71) Applicant: EMS S. A., 13186-901 Hortolândia - SP (BR); Universidade Estadual Paulista Julio De Mesquita Filho - UNESP, 01049 - 010 Sao Paulo - SP (BR)
(72) Inventor: SANTOS, Jean Leandro dos Santos, 14801-340 Araraquara - SP (BR); VIZIOLI, Ednir de Oliveira, 14800-000 Araraquara - SP (BR); CHIN, Chung Man, 14800-000 Araraquara - SP (BR); MENEGON, Renato Farina, 14800-000 Araraquara - SP (BR); BLAU, Lorena, 14800-000 Araraquara - SP (BR)
(74) Representative: Fischer, Michael Maria
(86) International application number: PCT/BR2010/000099
(87) International publication number: WO 2010/115252

(57) **Abstract**

The present invention relates to phthalimide derivatives of non-steroidal and/or TNF-α modulating anti-inflammatory compounds as well as the process of obtaining the so-called derivatives, pharmaceutical compositions containing such derivatives and their uses, including use in the treatment of inflammatory diseases, especially those related to chronic inflammatory processes, such as rheumatoid arthritis and intestinal inflammatory diseases (for instance, Chron's disease) and the use of the referred to pharmaceutical compositions as antipyretic, analgesic and platelet antiaggregating medications.

## Description

### Field of the Invention

This invention relates to phthalimide derivatives of non-steroidal anti-inflammatory and/or TNF-α, modulators, as well as the process of obtaining the so-called derivatives, the pharmaceutical compositions containing such derivatives and their uses, including use in the treatment of inflammatory diseases, especially those related to chronic inflammatory processes, such as rheumatoid arthritis and intestinal inflammatory diseases (for instance, Crohn's disease) and the use of the referred compositions as antipyretic, analgesic and platelet antiaggregating medicines.

### Basis of the Invention

Inflammation is fundamentally a protective response of the body triggered by physical, chemical and biological stimuli that can cause pain, swelling, and in some cases, lead to dysfunction of the organ or tissue (see EP1485127).

The inflammatory process can be divided into two phases: chronic and acute. The acute inflammatory response is an immediate and nonspecific response against the aggressive agent. The response is always qualitatively the same, regardless the stimulus. Chronic inflammation is always preceded by acute inflammation and is not observed at this stage, the characteristic signs of acute reactions (pain, tumor, warmth, redness and loss of function). It is characterized by an increase in the grade of cellularity and other tissue elements, given the permanence of the offending agent. Chronic inflammation can be subdivided into: nonspecific, productive (or hyperplasic), exudative and granulomatous. In general it is characterized by the presence of mononuclear macrophage system (lymphocytes, macrophages and plasma cells), tissue destruction caused by the presence of the offending agent and attempts at repair.

The use of medicinal substances to treat pain and fever has been reported since ancient civilizations that used plants with analgesic and anti-inflammatory potential. Literature reports point to the use of willow as an anti-inflammatory and analgesic prescribed by Hippocrates in the year 400 BC (Blobaum, A.L.; Marnett, L.J. Structural and functional basis of cyclooxygenase inhibition. J. Med. Chem. 50(7), p.1425-1441, 2007).

Currently, treatment for pain, inflammation and fever is carried out with anti-inflammatory drugs. These are among the most widely used of all therapeutic agents. The most widely used class of compounds is that of non-steroidal anti-inflammatory drugs (NSAIDs). It is estimated that there are more than 50 different NSAIDs on the market, but none is ideal in controlling or modifying the signs and symptoms of inflammation, particularly those that occur in common inflammatory joint disease (Rang, H.P.; Dale M.M.; Ritter, J.M. Pharmacology. 4 ed. Rio de Janeiro: Editora Guanabara Koogan. Cap. 13, p.189, 2001).

The action mechanism of NSAIDs was first deciphered by Vane, who observed a decrease in the concentration of prostaglandins in lung tissue treated with salicylates (Vane, J.R. Inhibition of prostaglandin synthesis as a mechanism of action for aspirin-like drugs. Nature (London) New Biol. 231, p. 232-235, 1971).

From Vane's studies, it was proposed that the mechanism of action of NSAIDs would be related to the inhibition of prostaglandin formation due to the ability of these compounds to inhibit enzyme cyclooxygenase (COX) that catalyzes the conversion of arachidonic acid into prostaglandins in the physiological and inflammatory process (Howard, P.; Delafontaine, P. Nonsteroidal Anti-Inflammatory Drugs and Cardiovascular Risk. Journal of the American College of Cardiology Vol. 43, No. 4, 2004).

In 1991, it was discovered the existence of two isoforms of cyclooxygenase (COX-1 and COX-2) with different expression profiles, but with a 60% homology among the aminoacid sequences (Kujubu, D. A.; Fletcher, B. S.; Varnum, B. C.; Lim, R. W.; Herschman, H. R. TIS10, a phorbol ester tumor promoter-inducible mRNA from Swiss 3T3 cells, encodes a novel prostaglandin synthase/cyclooxygenase homologue J. Biol. Chem. 266, 12866-12872, 1991).

COX-1 is a constitutive enzyme involved in several physiological processes in the body, such as for instance, mucus production responsible for protecting the gastric lining in the stomach acid environment. COX-2 can be induced in response to agents such as cytokines, mitogens, endotoxins of the inflammatory process (Sapirstein A., Saito H., Texel S.J., Samad T.A., O'Leary E., Bonventre J.V. Cytosolic phospholipase A2alpha regulates induction of brain cyclooxygenase-2 in a mouse model of inflammation. Am J Physiol Regul Integr Comp Physiol. 288 (6): p.1774-82, 2005). Initially, one would imagine that as induced isoform, COX-2 would be an important target for selected NSAIDs that would not interfere in the normal physiological process. Thus, we developed non-steroidal anti-inflammatory drugs selective inhibitors of COX-2 isoform, such as for instance, celecoxib that did not show the ability to cause gastric damage as the 1^{st} generation of NSAIDs.

However, afterwards, it was discovered that COX-2 plays an important physiological role in the brain, kidneys and cardiovascular system.

In the circulatory system, for example, the balance between pro- and anticoagulant activity is affected by the use of COX-2 selective drugs such as rofecoxib, for example. Thus the inhibition of COX-2 increases the risk of thrombus formation and deleterious cardiovascular events. This fact prompted the withdrawal of rofecoxib by Merck in September 2004 (Nartey, JL, Reichenbach, S., Sterchi R., Dieppe, P., Egger, M. Risk of cardiovascular events and rofecoxib: cumulative meta-analysis. The Lancet, Volume 364, Issue 9450, Pages 2021-2029, 2004; Howard & Delafontaine, 2004).

In the brain, the inhibition of prostaglandin synthesis has been reported as beneficial for the treatment of various diseases such as Alzheimer's and Parkinson's. It is said that NSAIDs have a neuroprotective action reducing the incidence and risk of Alzheimer's disease and significantly slows the disease progression (Colin K. Combs, Derrick E. Johnson, J. Colleen Karlo, Steven B. Cannady, and Gary E. Landreth Inflammatory Mechanisms in Alzheimer's Disease: Inhibition of β-Amyloidal-Stimulated proinflammatory Responses and Neurotoxicity by PPARg agonists. The Journal of Neuroscience. 20 (2):558-567, 2000; Blasko, I., Stampfer-Kountchev, M.; Robatscher, P.; Veerhuis, R., Eikelenboom, P.; Grubeck-Loebenstein, B. How chronic inflammation can affect the brain and support the development of Alzheimer's disease in old age: the role of microglia and astrocytes. Aging Cell. p.169-176, 2004).

Patients with rheumatoid arthritis being treated with NSAIDs had significantly reduced risks of presenting Alzheimer's disease (McGeer PL, McGeer EG Anti-inflammatory drugs in the fight against Alzheimer's disease. Ann NY Acad Sci 777:213-220, 1996; Stewart WF, Kawas C., Corrada M., Metter, EJ Risk of Alzheimer's disease and duration of NSAID use. Neurology 48:626-632, 1997).

The 1^{st} generation NSAIDs like diclofenac, ibuprofen, salicylic acetyl acid and naproxen inhibit COX-1 and COX-2. This non-selectivity makes these types of NSAIDs inhibit as well important prostaglandins of the physiological process responsible for instance, for gastric protection. That's why, every so often, the NSAIDs cause gastropathies such as ulcers and bleeding (Steen, KSS; Nurmohamed, MT; Visman, I.; Heijerman, M.; Boers, M.; Dijkmans, BAC; Lems, WF Decreasing incidence of symptomatic gastrointestinal ulcers and ulcer complications in patients with rheumatoid arthritis. Ann Rheum Dis. 67:256 - 259, 2008). Furthermore, some chronic inflammatory illnesses as, for example, rheumatoid arthritis, Chron's disease and osteoarthritis demand daily administration of NSAIDs, which increases even more the probability of ulcer formation. Several studies have shown an increased risk of developing ulcers due to NSAID use (Ong, CKS; Lirk, P., Tan, CH, Seymour, RA An Evidence-Based Update on Nonsteroidal Anti-Inflammatory Drugs. Clinical Medicine & Research.5 (1), p. 19-34, 2007; Babush, J.G.; Tripp, M.L.; Howell, T.; Bland, J.S.; Darlan, G.; Lerman, R.; Lukaczer, D.O. Anti-inflammatory pharmaceutical compositions for Reducing inflammation and the Treatment or prevention of gastric toxicity. WO 2005/039483 A2. 06/05/2005; Wallace, J.L., Keenan, C.M., Granger, D.N. Gastric ulceration induced by nonsteroidal anti-inflammatory drugs is a neutrophyl-dependent process. Am J Physiol. 259(3):462-467; Armstrong, C.P, Blower, A.L. Non-steroidal anti-inflammatory drugs and life threatening Complications of peptic ulceration. Gut. 28(5): 527-532, 1987).

Besides gastric ulceration, other adverse effects have been related to the use of NSAIDs, among them: skin lesions, acute renal failure, chronic nephritis, renal papillary necrosis, liver disorders, bone marrow disorders (Ong, C.K.S.; Lirk, P.; Tan, C.H.; Seymour, R.A. An Evidence-Based Update on Nonsteroidal Anti-Inflammatory Drugs. Clinical Medicine & Research.5 (1), p. 19-34, 2007).

Currently, other targets of the inflammatory process have been explored and the control of the inflammatory process by inhibiting distinct pathways acts synergistically in controlling inflammation. During the inflammatory process we have the participation of various substances including: prostaglandins, cytokines, histamine, serotonin and others. Among the pro-inflammatory cytokines we can highlight: YL-1β, YL-6, YL-8 and TNF-α, (Kraychete, D.C.; Calasans, M.T.A.; Valente, C.M.L. Pro-inflammatory Cytokines and Pain. Rev Bras Reumatol, v. 46, n.3, p. 199-206, 2006).

TNF-α is produced by many cell types including monocytes and macrophages, T and B lymphocytes, neutrophyls, tumor cells and fibroblasts. TNF-α is the key regulator of other proinflammatory cytokines including interleukin 1-β (YL-1β, YL-6 and YL-8). In physiological conditions, low grades of TNF-α confer protection against infectious agents, tumor and tissue damage. However, overproduction of these cytokines can lead to the development of autoimmune diseases, inflammatory diseases and immunopathological diseases (Alexandre-Moreira, M.S.; Takiyac, C.M.; Arrudad, L.B.; Pascarellic, B.; Gomese, R.N.; Neto, H.C.C.F.; Lima, L.M.; Barreiro, E.J. LASS Bio-468: a new achiral thalidomide analogue which modulates TNF-α and β production and inhibits endotoxic shock and arthritis in an animal model. International Immunopharmacology. 5, p.485-494, 2005).

TNF-α exerts proinflammatory effects by increasing chemotactic properties, the adhesion of neutrophyls to the endothelium due to increased molecules adhesion, stimulating the production of free radicals and the synthesis of other inflammatory mediators such as YL-1 and PGE2. TNF-α also induces changes in coagulant and anticoagulant properties and increases the liver synthesis of a few acute phase reagents. Moreover, it is an important mediator of sepsis syndrome and endotoxic shock, being able to suppress the biosynthesis of lipoprotein lipase and lipogenic enzymes in adipose tissue, damaging lipid storage in adipocytes.

The ability of the TNF-α to change the anticoagulant properties of vascular endothelium and induce procoagulant activity on endothelial cell surface, stimulating the production of platelet activating factor (PAF), and increasing leukocyte adhesion to vascular endothelial cells, results in the increase of the resistance to blood flow, making it more difficult for the circulation and, therefore, exacerbating microvascular stasis.

Several substances have been reported as having a direct effect on the inhibition of TNF-α. Among them are converting enzyme inhibitors of tumor necrosis factor-alpha (TNF-α), neutralizing antibodies (infliximab), and drugs structurally related to thalidomide (Klotz, U.; Teml, A.; Schwab, M. Clinical Pharmacokinetics and Use of Infliximab. Clin Pharmacokinet 2007; 46 (8): 645-660; Lima, L.M.; Fraga, C.A.M.; Barreiro, E.J. The rebirth of a drug: thalidomide. Quim. Nova. v.24. n.5, p.683-688, 2001). Thalidomide, with its phthalmide structure has been described for the first time in document GB768821.

It has also been reported that arthritic patients treated with monoclonal antibodies anti-TNF-α (infliximabe) presented a decrease of the pain associated with the disease (Rankin, E.C., Choy, E.H., Kassimos, D., Kingsley, G.H., Sopwith, A.M., Insenberg, D.A., Panayi, G.S. The therapeutic effects of an engineered human anti-tumor necrosis factor alpha antibody_CDP571 in rheumathoid arthritis. Br. J. Rheumatol. 34, 334-342, 1995). This fact demonstrates that the TNF-α has an important role in peripheral hiperalgesia, and its inhibition has been associated to the reduction of acute and chronic pain, what explains the analgesic effect of the thalidomide one, first drug anti-TNFα introduced in treatment (Ribeiro, R.A.; Vale, M.L.; Ferriera, S.H.; Cunha, F.Q. Analgesic effect of thalidomide on inflammatory pain. Eur. J. Pharmacol., v.391, p.97-103, 2000).

Several laboratories and research groups have reported the anti-inflammatory and immunomodulatory properties of thalidomide, demonstrating its therapeutic potential against the treatment of pathologies such as multiple myeloma, cachexia, tuberculosis, arthritis, sickle cell anemia among others (Miyachi, H.; Ogasawara, A.; Azuma, A.; Hashimoto, Y.). Tumor necrosis factor-alpha production-inhibiting activity of phtalimide analogues on human leukemia THP-1 cells and a structure-activity relationship study. Bioorg. Med. Chem. v.5, n. 11, p.2095-2102, 1997; Lima, 2001).

The development of new thalidomide analogues containing the key pharmacophore for inhibiting activity of TNF, and devoid of toxicophore groups responsible for teratogenicity is the ultimate goal for the development of new therapeutic possibilities for the treatment of pathologies associated with or exacerbated by increased plasma grades of TNF.

It has also been reported that patients with chronic inflammatory diseases have high grades of proinflammatory cytokine TNF-α. The high grade of this cytokine triggers a cascade of harmful changes because it exacerbates the inflammatory process (Suryaprasad, AG, Prindiville T. The biology of TNF blockade). Autoimmunity Reviews. 2, 346-357, 2003; Popa, C., Netea M.G., van Riel P.L.C.M., van der Meer J.W.M. and Stalenhoef A.F.H. The role of TNF-α in chronic inflammatory conditions, intermediary metabolism, and cardiovascular risk. J. Lipid Res. 48: 751-762, 2007; Clark, A.I. How TNF was recognized as a key mechanism of disease. Cytokine & Growth Factor Reviews. 18, 335-343, 2007; Kodamaa, S., Davis, M. and Faustman, D. L. The therapeutic potential of tumor necrosis factor for autoimmune disease: a mechanistically based hypothesis. Cell. Mol. Life Sci. 62, 1850-1862, 2005; Aggarwal, B.B.; Shishodia, S.; Ashikawa, K.; Bharti, A.C. The Role of TNF and Its Family Members in Inflammation and Cancer: Lessons from Gene Deletion Current Drug Targets - Inflammation & Allergy. 1, 327-341, 2002; Koch, A.A.; Zacharowski, K.; Boehm, O.; Stevens, M.; Lipfert, P.; Giesen, H.J.V.; Wolf, A.; Freynhagen, R. Nitric oxide and pro-inflammatory cytokines correlate with pain intensity in chronic pain patients. Inflammation Research. 56, p. 32-37, 2007; Schenk, M.; Bouchon, A.; Seibold, F.; Mueller, C. TREM-1-expressing intestinal macrophages crucially amplify chronic inflammation in experimental colitis and inflammatory bowel diseases. J. Clin. Invest. 117:3097-3106, 2007).

Thus, the strategy of inhibiting TNF-α is useful for treating diseases such as rheumatoid arthritis, Chron's disease, ulcerative colitis, uveitis, osteoarthritis, psoriasis, psoriatic arthritis, sickle cell disease, leprosy and several other diseases in which there is increased plasma grades of this kytocine (Valesini, G.; Iannuccelli, C.; Marocchi, E.; Pascoli, L.; Scalzi, V.; Di Franco, M. Biological and clinical effects of anti-TNFα treatment. Autoimmunity Reviews. 7, 35-41, 2007; Williams, R.O.; Paleolog, E.; Feldmann, M. Cytokine inhibitors in rheumatoid arthritis and other autoimmune diseases. Current Opinion in Pharmacology. 7:412-417, 2007; Alexandre-Moreira et al 2005).

Among the strategies used to introduce a new drug in the therapy, the molecular modification has shown promising. This helps in the discovery of new drugs with more adequate pharmacokinetic and pharmacodynamic profile than the original prototype. Among the techniques of molecular modification the following can be cited: latentiation, bioisosterism, hybridization, homology, annelation among other (Wermuth, C.G. Selective optimization of side activities: another way for drug discovery. J Med Chem. v.47, n.6, p.1303-1312, 2004).

The use of molecular modification, more specifically molecular latentiation and hybridization has allowed the acquisition of active anti-inflammatory compounds and with less adverse effects, particularly lower rates of gastroulceration.

Cena et al (Cena, C.; Lolli, M.L.; Lazzarato, L.; Guaita, E.; Morini,G.; Coruzzi, G.; McElroy, S.P.; Megson, I.L.; Fruttero, R.; Gasco, A. Anti-inflammatory, Gastrosparing, and Antiplatelet Properties of New NO-Donor Esters of Aspirin. J. Med. Chem. 46, p.747-754, 2003) using the molecular hybridization strategy to obtain hybrid compounds of NSAIDS with furoxanic subunit, nitric oxide donor, with potential anti-inflammatory, analgesic and anti-platelet activities.

The preparation of new compounds with anti-inflammatory and analgesic activity to reduce the adverse effects of NSAIDs is a need for treatment of pain and inflammation. The new compounds described are obtained by molecular hybridization strategy between the phthalmidic subunit of thalidomide (held responsible for the inhibition of TNF-α) and NSAIDs. Through this strategy, it is possible to act synergistically in chronic inflammatory diseases on two fronts: a front by inhibiting cyclooxygenase (COX), responsible for the conversion of arachidonic acid into prostaglandins, and other front inhibiting alpha tumor necrosis factor, responsible for amplification of the inflammatory response. Thus, new compounds of the invention present therapeutic advantages in that they act on different targets in the same disease and constitute a new therapeutic approach for treating chronic inflammatory diseases.

### Summary of the Invention

This invention aims at providing a differentiated alternative for the treatment of chronic inflammatory diseases. More specifically, the new NSAID's phthalimide derivatives act on such diseases potentiating the anti-inflammatory and analgesic activity by acting on different targets: inhibition of cyclooxygenase (responsible for the production of prostaglandins) and inhibition/modulation of the tumor necrosis factor-alpha (TNF-α). The inhibition and/or modulation of these different targets synergistically assist in controlling the inflammatory process and constitute a new approach for the treatment of various diseases such as, for instance, rheumatoid arthritis and Chron's disease.

The invention is aimed at minimizing the major limitations and complications associated with drug therapy, especially that based on NSAIDs, that is, provide an alternative to reduce side and adverse effects and of compounds usually used in the treatment of chronic inflammatory processes through the use of compounds of the invention, such as inhibitors and/or modulators of TNF-α and enzyme cyclooxygenase (COX), improving the patient's life quality. Thus, one of the main adverse effects of the anti-inflammatory drugs of first generation, which is the gastroculceration, can be markedly decreased, justifying their use in the treatment of chronic pain/inflammation. In addition, due to the higher lipophilicity of new derivatives, they can act as anti-inflammatory in the central nervous system, aiding in the treatment of some diseases such as, for example, Alzheimer's and Parkinson's.

A first substantiation of the invention relates to phthalimide derivatives of functioning non-steroidal anti-inflammatory compounds having the general formula (I) 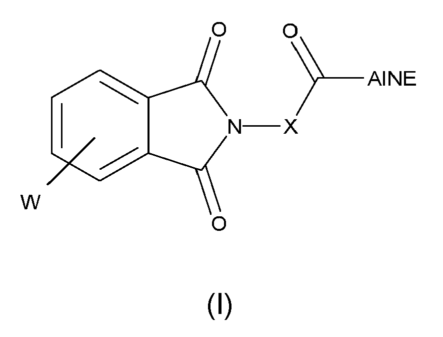 Where:
- NSAID represents a non-steroidal anti-inflammatory compound containing at least one carboxylic acid subunit, including diclofenac, lumiracoxib, naprofen, ibuprofen, salicylic acid, mesalamine, olsalazine, indomethacin, aceclofenac and ketoprofen;
- W represents one or more independently selected substituents from the group consisting of F, Cl, Br, NO₂, NH₂, OH, OCH₃, OCF₃, CF₃, CH₃; and
- X represents - (CH₂) ₙO-, Where n = 0, 1, 2, 3, 4, 5, 6; 2-hydroxy-phenyl, 3-hydroxy-phenyl, 4-hydroxy-phenyl, 2-hydroxy-benzyl, 3-hydroxy-benzyl, 4-hydroxy-benzyl, 2-hydroxy-ethylbenzyl, 3-hydroxy-ethylbenzyl, 4-hydroxy-ethylbenzyl, benzyl, thyophene, furan, pyrrole, 2-hydroxy-pyridine, 3-hydroxy-pyridine, 4-hydroxy-pyridine, pyrazine, pyrimidine, benzothiophene, benzofuran, indole, quinoline, isoquinoline, naphthalene, CH₂-2-hydroxy-thyophene, CH₂-3-hydroxy-thyofene, CH₂-2-hydroxy-furan, CH₂-3-hydroxy-furan, CH₂CH₂-2-hydroxy-thyophene, CH₂CH₂-3-hydroxy-thyophene, CH₂CH₂-2-hydroxy-furan, CH₂CH₂-3-hydroxy-furan; 2-amino-phenyl, 3-amino-phenyl, 4-amino-phenyl, 2-amino-benzyl, 3-amino-benzyl, 4-amino-benzyl, 2-amino-ethylbenzyl, 3-amino-ethylbenzyl, 4-amino-ethylbenzyl, benzyl, thyophene, furan, pyrrole, 2-amino-pyridine, 3-amino-pyridine, 4-amino-pyridine, pyrazine, pyrimidine, benzothiophene, benzofurans, indole, quinoline, isoquinoline, naphthalene, CH₂-2-amino-thyophene, CH₂-3-amino-thyophene, CH₂-2-amino-furan, CH₂-3-amino-furan, CH₂CH₂-2-amino-thyofene, CH₂CH₂-3-amino-thyophene, CH₂CH₂-2-amino-furan, CH₂CH₂-3-amino-furan.

The compounds of the invention, included within the possibilities of Formula I are useful as active ingredients in pharmaceutical compositions, both in the quality of the drugs as prodrugs.

In a second substantiation of the invention a method is provided to obtain functioning phthalimide derivatives of non-steroidal anti-inflammatory compounds having the general Formula (I) including the stages of: (i) esterification reaction of NSAID by acid catalysis exploring the nucleophilicity of methanol; (ii) nucleophilic substitution by hydrazine or condensation of hydrazine with carboxylic acid of NSAID using coupling agents to obtain the hydrazides; (iii) condensation of hydrazides duly functioning to phthalimide anhydride in an appropriate solvent, and (iv) separation of the functioning phthalimide derivative (iv) separation of the functioning desired phthalimide derivative among the compounds estimated in the general Formula (I). Preferably, the suitable solvent used in stage (iii) is selected from the group consisting of acetic acid and pyridine. The products resulting from this process are of hybrid character that is usable in pharmaceutical compositions such as pharmaceuticals.

A third substantiation of the invention relates to an alternative process to obtain compounds having the general Formula I, said process comprising the stages of: i) preparation of the phthalimide compounds properly functionalized, as structure represented in the first substantiation, (ii) condensation of the phthalimide derivatives with carboxylic acid of the anti-inflammatory agents using coupling agents in inert solvents, and (iii) separation of the desired functionalized phthalimide derivative from the compounds estimated in the general Formula (I). The products resulting from this process are of hybrid character that is usable in pharmaceutical compositions such as prodrugs.

In a fourth substantiation of the invention, the process for obtaining compounds having the general Formula I comprises a preliminary stage of halogenations of carboxylic acids of NSAIDs with halogenizing agents preferably represented by oxalyl chloride or thionyl chloride to obtain acyl halides, being the resulting acyl halides submitted to a condensation reaction with the phthalimide derivates duly functionalized in inert solvents to obtain the compounds of general Formula I. Preferably, esters and amides are obtained, estimated in Formula I and that are not usable in pharmaceutical compositions as prodrugs.

A fifth substantiation of the invention relates to a pharmaceutical composition comprising: (a) a therapeutically effective amount of at least one phthalimide derivative of non-steroidal anti-inflammatory compounds functionalized with the general Formula (I) and (b) a pharmaceutically acceptable carrier. Additionally, the composition of the invention may also contain one or more drugs suitable for use in treating medical conditions involving pain and associated inflammation treatment.

In a sixth substantiation, the invention is directed to the use of a phthalimide derivative of non-steroidal anti-inflammatory compounds functionalized having the general formula (I) of the invention in medical conditions involving pain and inflammation.

### Brief Description of the Figures:

Figure 1 illustrates the anti-inflammatory evaluation of the compound prepared according to Example 1.3 through the model of rat paw edema induced by carrageenan administered orally in a single dose of 100 µmol/kg.
Figure 2 illustrates the anti-inflammatory evaluation of the compound prepared according to Example 2.3 through the model of rat paw edema induced by carrageenan administered orally in a single dose of 100 µmol/kg.
Figure 3 illustrates the anti-inflammatory evaluation of the compound prepared according to Example 3.3 through the model of rat paw edema induced by carrageenan administered orally in a single dose of 100 µmol/kg.
Figure 4 illustrates the anti-inflammatory evaluation of the compound prepared according to Example 5.3 through the model of rat paw edema induced by carrageenan administered orally in a single dose of 100 µmol/kg.
Figure 5 illustrates the anti-inflammatory evaluation of the compound prepared according to Example 6.3 through the model of rat paw edema induced by carrageenan administered orally in a single dose of 100 µmol/kg.
Figure 6 shows pictures of the stomachs of rats treated with phthalimide derivatives of the invention administered at a single dose of 100 µmol/kg and saline showing no gastric ulceration.
Figure 7 shows photographs of the stomachs of rats treated with diclofenac (grade of injury = 5) or celecoxib (grade of injury = 2) showing their ulcers caused by administration of the anti-inflammatory drug in a single dose at the dose of 100 µmol/kg.
Figure 8 shows the percentage of inhibition of abdominal constrictions induced by acetic acid 0.1 N in the presence of the hybrid compounds of the invention at concentrations of 1 µmol/kg and 100 µmol/kg (5% gum Arabic - control - showed no analgesic activity).

### Detailed Description of the Invention

This invention relates to a process for obtaining phthalimide derivatives of non-steroidal anti-inflammatory compounds with anti-inflammatory, TNF-α-modulating, analgesic and antiplatelet properties useful in the treatment of inflammatory processes, especially those related to chronic inflammation, such as rheumatoid arthritis and Chron's disease. Even more particularly, the process of obtaining these compounds, and the use of these in pharmaceutical compositions and medications are described.

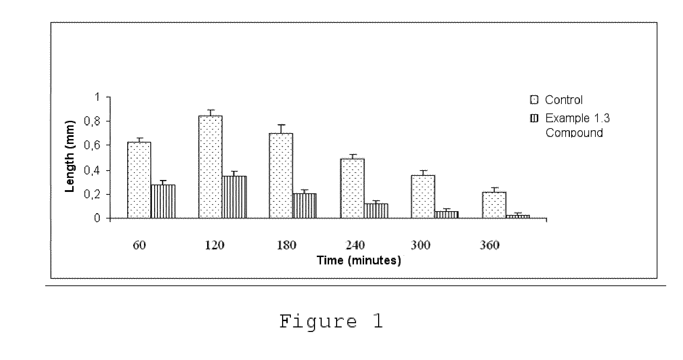

The invention has as one of the innovative features the synthesis of new anti-inflammatory derivatives, designed from the prototype anti-inflammatory drugs (1) and compounds with phthalimide structure, such as thalidomide (2) and rationally planned through the molecular hybridization strategy in order to obtain drugs with dual inhibiting action of cyclooxygenase and TNF-α modulator with improved anti-inflammatory and/or analgesic activity, for instance, the activity illustrated in Figure 1 of the compound obtained from Example 1.3.

Examples of NSAIDs preferred phthalimide derivatives of this invention are presented in Table 1.

**Table 1: Examples of NSAIDs preferred phthalimide derivatives of the invention.**

| | | |
|---|---|---|
| 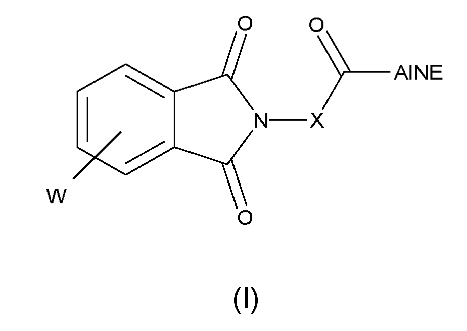 | | |

| **Non-steroid Anti-inflammatory drug (NSAID)** | X | **Compound of the invention** |
|---|---|---|
| | NH | 2 - (2 - (2,6-dichlorophenylamine) phenyl)-N-(1,3-dioxyisoindolin-2-yl) acetamide |
| 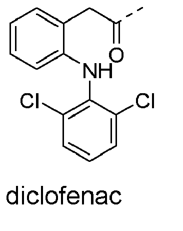 | | 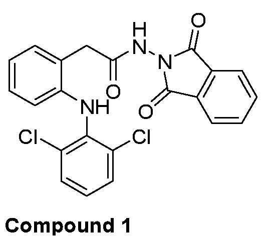 |
| | -CH₂O- | 2 - (1,3-dioxyisoindolin-2-yl) methyl 2 - (2 - (2,6-dichlorophenylamino) phenyl) acetate |
| 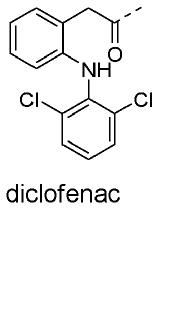 | | 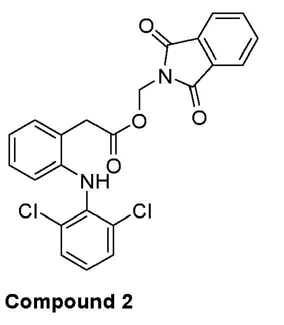 |
| | -CH₂CH₂O- | 2 - (1,3-dioxyisoindolin-2-yl) ethyl 2 - (2 - (2,6-dichlorophenylamine) phenyl) acetate |
| 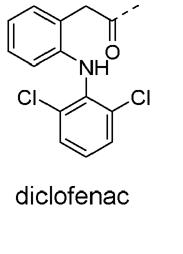 | | 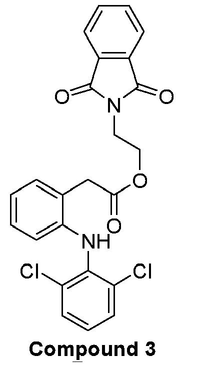 |
| | 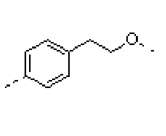 | (4 - (1,3-dioxyisoindolin-2-yl) phenyl) ethyl 2 - (2 - (2,6-dichlorophenylamine) phenyl) acetate |
| 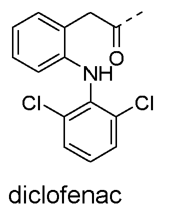 | | 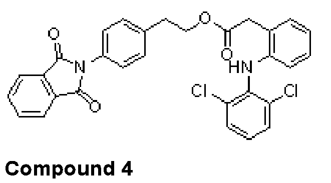 |
| | 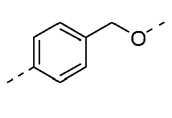 | 4 - (1,3-dioxyisoindolin-2-yl) benzyl 2 - (2 - (2,6-dichlorophenylamine) phenyl) acetate |
| 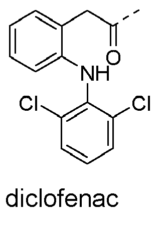 | | 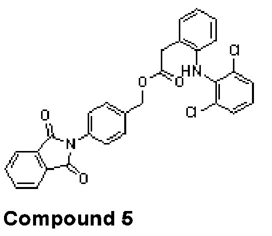 |
| | 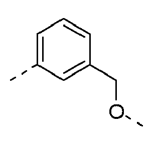 | 3 - (1,3-dioxyisoindolin-2-yl) benzyl 2 - (2 - (2,6-dichlorophenylamine) phenyl) acetate |
| 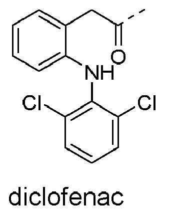 | | 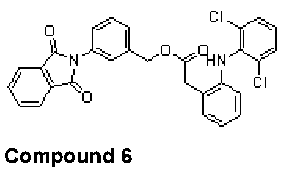 |
| | 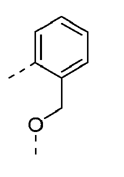 | 2 - (1,3-dioxyisoindolin-2-yl) benzyl 2 - (2 - (2,6-dichlorophenylamine) phenyl) acetate |
| 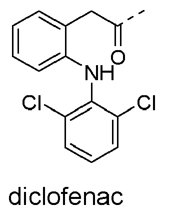 | | 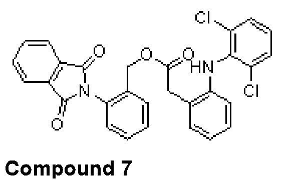 |
| | NH | 2 - (2 - (2-chloro-6-fluorphenylamine)-5-methyl-phenyl)-N-(1,3-dioxyisoindolin-2-yl) acetamide |
| 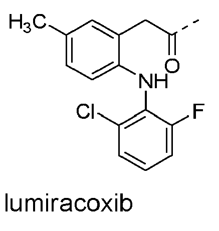 | | 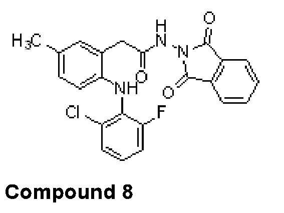 |
| | -CH₂O- | (Dioxyisoindolin-1.3-2-yl) methyl 2 - (2 - (2-chloro-6-fluorophenylamine)-5methylphenyl) acetate |
| 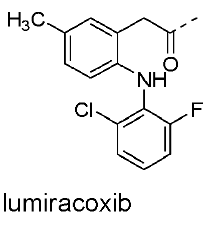 | | 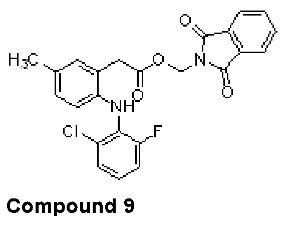 |
| | -CH₂CH₂O- | (1,3-dioxyisyindolin-1.3-2-yl) ethyl 2 - (2 - (2-chloro-6-fluorophenylamine)-5methylphenyl) acetate |
| 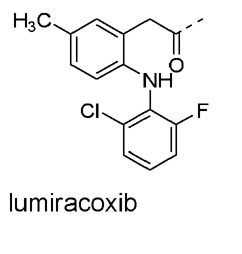 | | 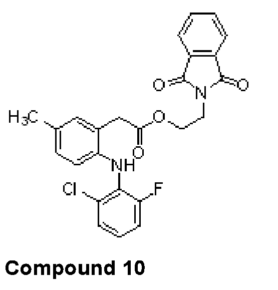 |
| | 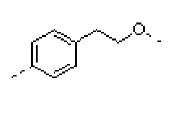 | 2 - (4 - (1,3-dioxyisoindolin-2-yl) phenyl) ethyl 2 - (2 - (2-chloro-6-fluorophenylamine)-5-methylphenyl) acetate |
| 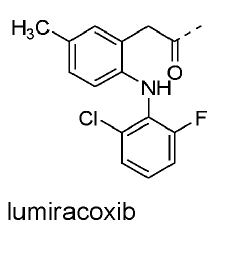 | | 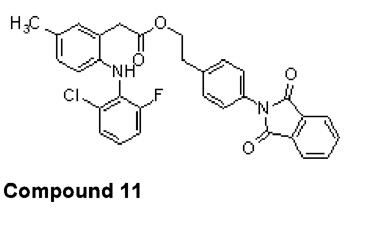 |
| | 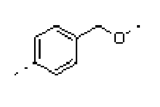 | (4 - (1,3 - (dioxyisoindolin-2-yl) phenyl) methyl 2 - (2 - (2-chloro-6-fluorophenylamine)-5-methylphenyl) acetate |
| 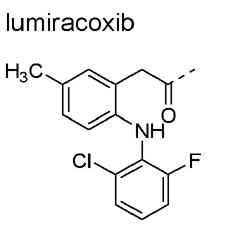 | | 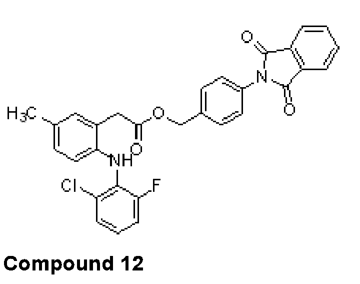 |
| | 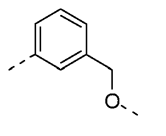 | (3 - (1,3 - (dioxyisoindolin-2-yl) phenyl) methyl 2 - (2 - (2-chloro-6-fluorophenylamine)-5-methylphenyl) acetate |
| 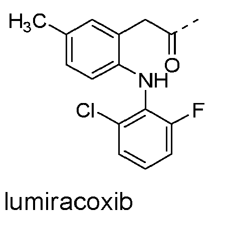 | | 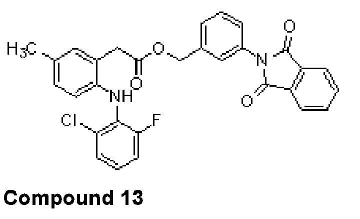 |
| | 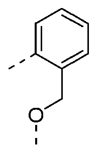 | (2 - (1,3 - (dioxyisoindolin-2-yl) phenyl) methyl 2 - (2 - (2-chloro-6-fluorophenylamine)-5-methylphenyl) acetate |
| 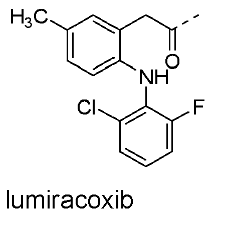 | | 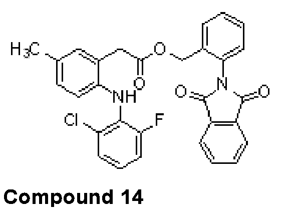 |
| | NH | 2 - (2-methoxynaphthalene-6-yl)-N-(1,3-dioxyisoindolin-2-yl) propanamide |
| 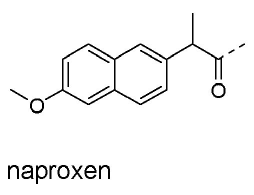 | | 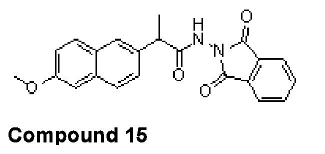 |
| | -CH2O- | (1,3-dioxyisoindolin-1.3-2-yl) methyl 2 - (2-methoxynaphthalene-6-yl) propanoate |
| 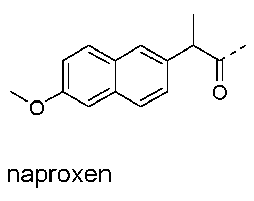 | | 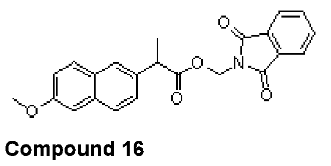 |
| | -CH₂CH₂O- | (1,3-dioxyisoindolin-1.3-2-yl) ethyl 2 - (2-methoxynaphthalene-6-yl) propanoate |
| 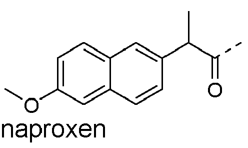 | | 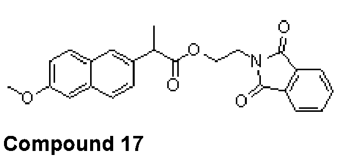 |
| | 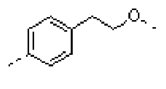 | 2 - (4 - (1,3-dioxyisoindolin-2-yl) phenyl) ethyl 2 - (2-methoxynaphthalene-6-yl) propanoate |
| 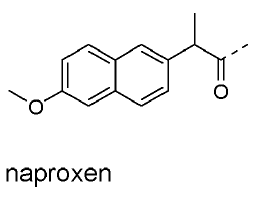 | | 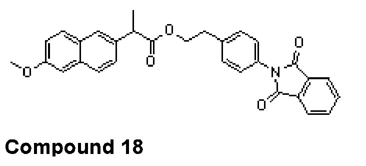 |
| | 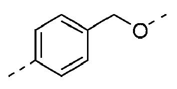 | 2 - (4 - (1,3-dioxyisoindolin-2-yl) phenyl) ethyl 2 - (2-methoxynaphthalene-6-yl) propanoate |
| 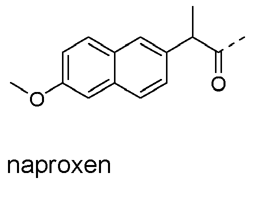 | | 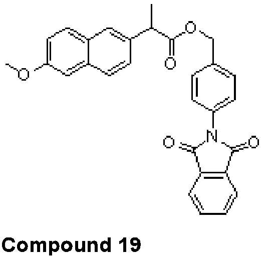 |
| | 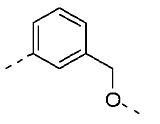 | (3 - (1,3-dioxyisoindolin-2-yl) phenyl) methyl 2 -(2-methoxynaphthalene-6-yl) propanoate |
| 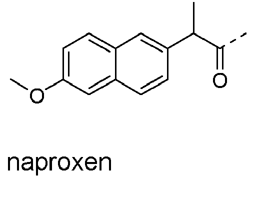 | | 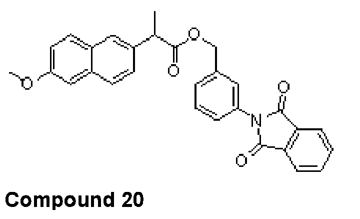 |
| | 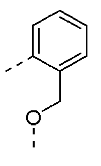 | (3 - (1,3-dioxyisoindolin-2-yl) phenyl) methyl 2 -(2-methoxynaphthalene-6-yl) propanoate |
| 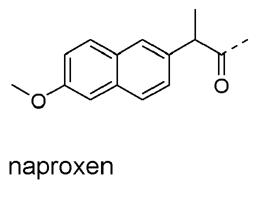 | | 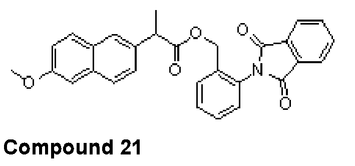 |
| | NH | 2-hydroxy-N-(1,3-dioxyisoindolin-2-yl) benzamide |
| 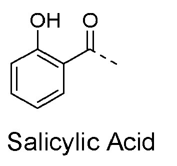 | | 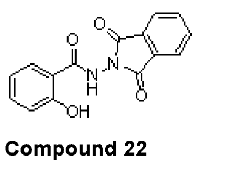 |
| | -CH2O- | 2 - (1,3-dioxyisoindolin-2-yl) methyl 2-hydroxybenzoate |
| 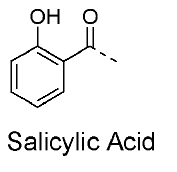 | | 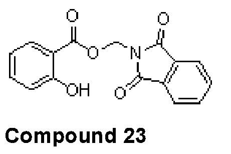 |
| | -CH₂CH₂O- | 2 - (1,3-dioxyisoindolin-2-yl) ethyl 2-hydroxybenzoate |
| 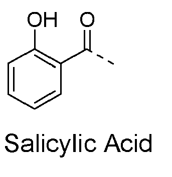 | | 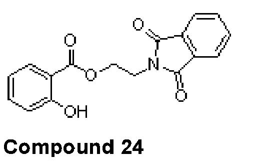 |
| | 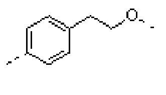 | 2 - (4 - (1,3-dioxyisoindolin-2-yl) phenyl) ethyl 2-hydroxybenzoate |
| 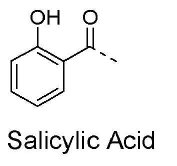 | | 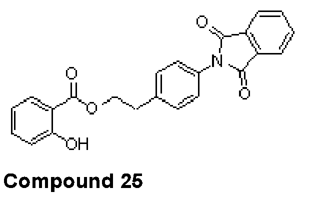 |
| | 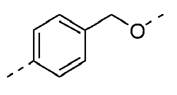 | 4 - (1,3-dioxyisoindolin-2-yl) phenyl) methyl 2-hydroxybenzoate |
| 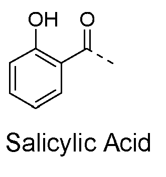 | | 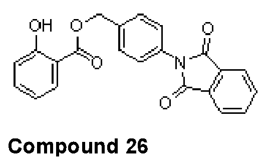 |
| | 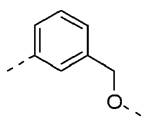 | 3 - (1,3-dioxyisoindolin-2-yl) phenyl) methyl 2-hydroxybenzoate |
| 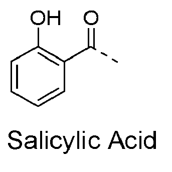 | | 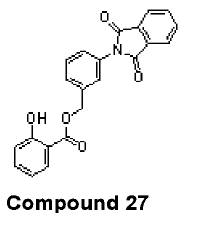 |
| | 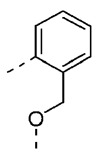 | 2 - (1,3-dioxyisoindolin-2-yl) phenyl) methyl 2-hydroxybenzoate |
| 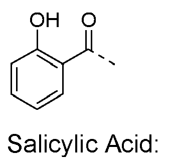 | | 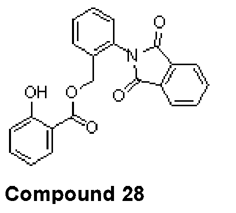 |
| | -NH- | 2 - (4-isobutylphenyl)-N-(1,3-dioxyisoindolin-2-yl) propanamide |
| 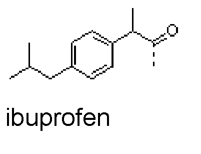 | | 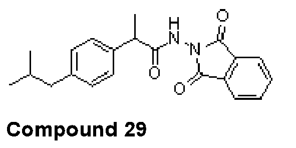 |
| | -CH₂O- | (1,3-dioxyisoindolin-1.3-2-yl) methyl 2 - (4-isobutylphenyl) propanoate |
| 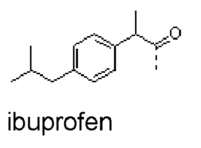 | | 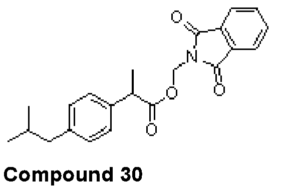 |
| | -CH₂CH₂O- | 2 - (1,3-dioxyisoindolin-2-yl) ethyl 2 - (4-isobutylphenyl) propanoate |
| 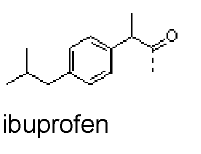 | | 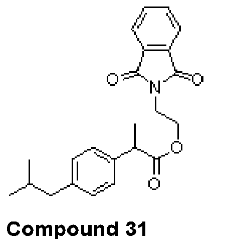 |
| | 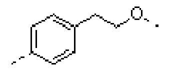 | 2 - (4 - (1,3-dioxyisoindolin-2-yl) phenyl) ethyl 2 - (4-isobutylphenyl) propanoate |
| 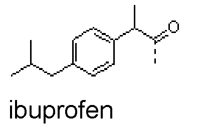 | | 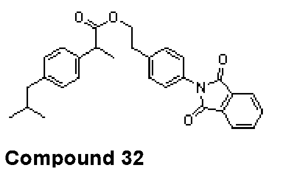 |
| | 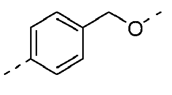 | 4 - (1,3-dioxyisoindolin-2-yl) phenyl) methyl 2 (4-isobutylphenyl) propanoate |
| 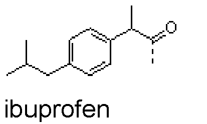 | | 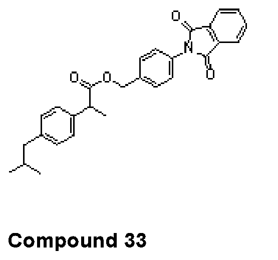 |
| | 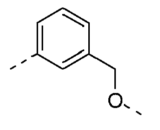 | 3 - (1,3-dioxyisoindolin-2-yl) phenyl) methyl 2 (4-isobutylphenyl) propanoate |
| 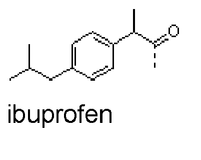 | | 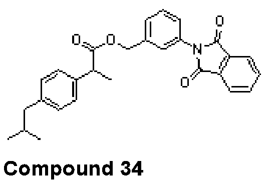 |
| | 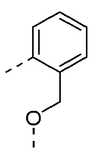 | 2 - (1,3-dioxyisoindolin-2-yl) phenyl) methyl 2 (4-isobutylphenyl) propanoate |
| 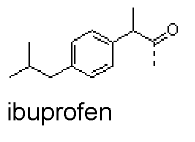 | | 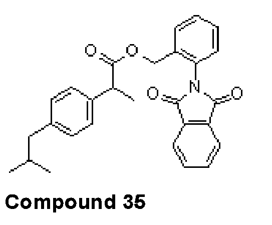 |
| | -NH- | 2- (3-benzoylphenyl)-N-(1,3-dioxy-1 ,3-dihydro-2H-isoindol-2-yl) propanamide |
| 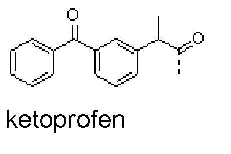 | | 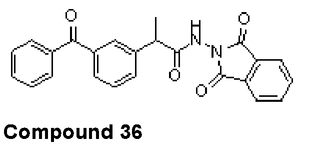 |
| | -CH₂O- | (1,3-dioxy-1,3-dihydro-2H-isoindolin-2-yl) methyl 2 - (3-benzoylphenyl) propanoate |
| 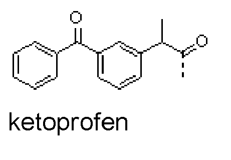 | | 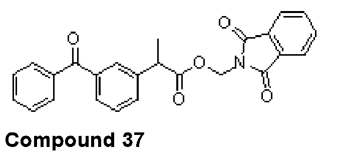 |
| | -CH₂CH₂O- | (1,3-dioxy-1,3-dihydro-*2H*-isoindol-2-yl) ethyl2-(3-benzoylpphenyl) propanoate |
| 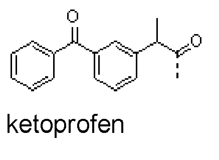 | | 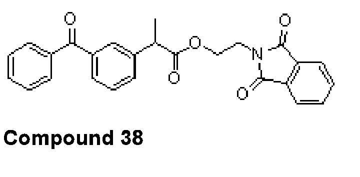 |
| | 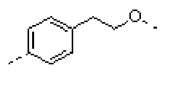 | 4 - (1,3-dioxyisoindolin-2-yl) phenyl) ethyl 2 - (3-benzoylphenyl) propanoate |
| 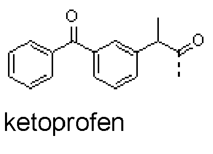 | | 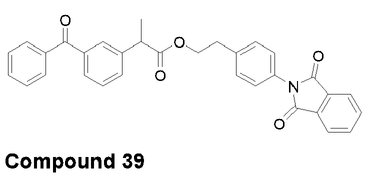 |
| | 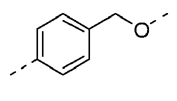 | (4 - (1,3-dioxyisoindolin-2-yl) phenyl) methyl 2 -(3-benzoylphenyl) propanoate |
| 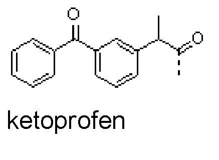 | | 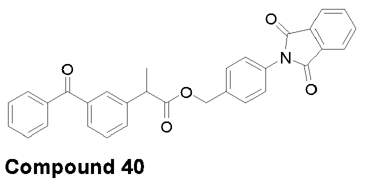 |
| | 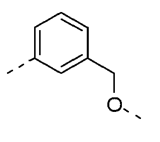 | (3 - (1,3-dioxyisoindolin-2-yl) phenyl) methyl 2 -(3-benzoylphenyl) propanoate |
| 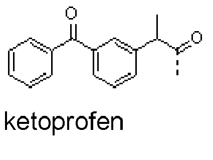 | | 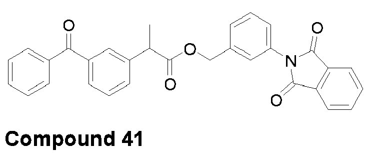 |
| | 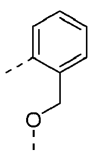 | -(1,3-dioxyisoindolin-2-yl) phenyl) methyl 2 - (3-benzoylphenyl) propanoate |
| 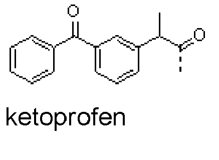 | | 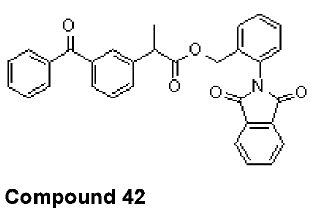 |
| | -NH- | 2 - [1 - (4-chlorobenzoyl)-5-methoxy-2-methyl-1H-indole-3-yl] acetohydrazidr-N-(1,3-dioxyisoindolin-2-yl |
| 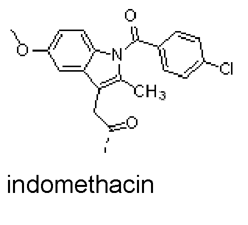 | | 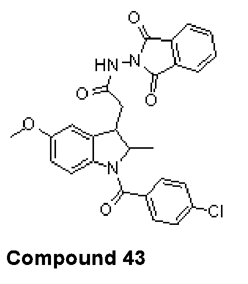 |
| | -CH₂O- | (1,3-dioxy-1 ,3-dihydro-*2H*-isoindol-2-yl) methyl [1 - (4-chlorobenzoyl)-5-methoxy-2-methyl-1H-indole-3-yl] acetate |
| 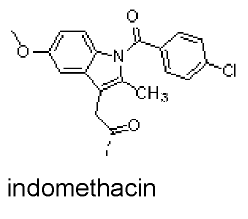 | | 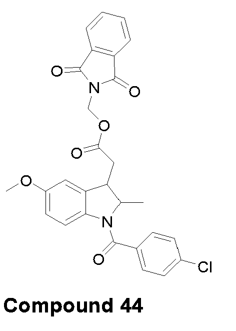 |
| | -CH₂CH₂O- | (1,3-dioxy-1,3-dihydro-*2H*-isoindol-2-yl) ethyl [1 - (4-chlorobenzoyl)-5-methoxy-2-methyl-1*H*-indole-3-yl] acetate |
| 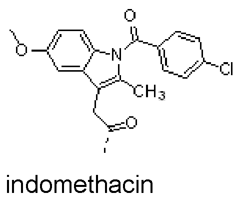 | | 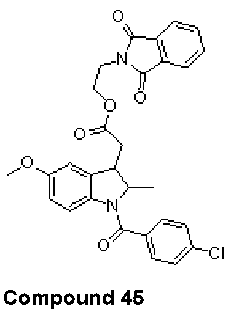 |
| | 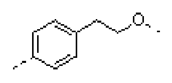 | (4 - (1,3-dioxyisoindolin-2-yl) phenyl) ethyl [4 -(4-chlorobenzoyl)-5-methoxy-2-methyl-1 H-indole-3-yl] acetate |
| 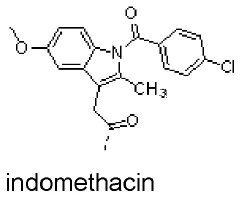 | | 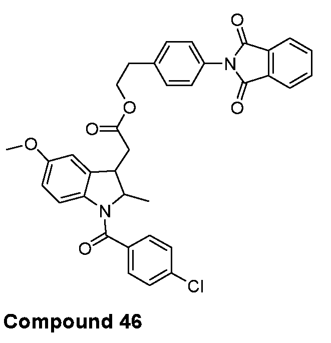 |
| | 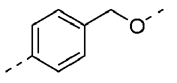 | (4 - (1,3-dioxyisoindolin-2-yl) phenyl) methyl [1 -(4-chlorobenzoyl)-5-methoxy-2-methyl-1*H*-indole-3-yl] acetate |
| 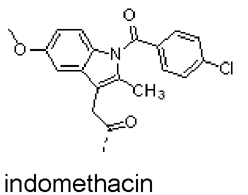 | | 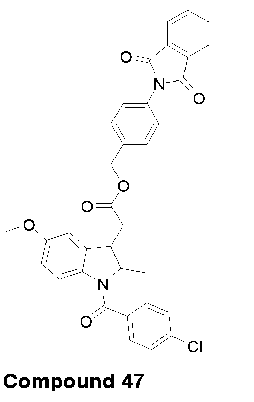 |
| | 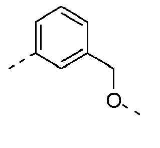 | (3-(1,3-dioxyisoindolin-2-yl)phenyl)methyl [1-(4-chlorobenzoyl)-5-methoxy-2-methyl-1*H*-indol-3-yl]acetate |
| 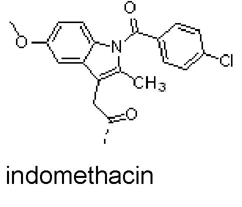 | | 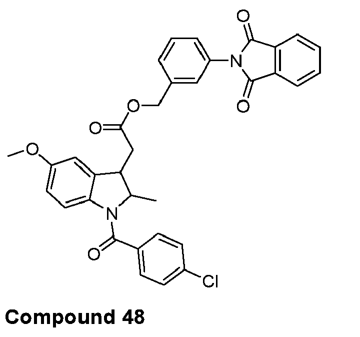 |
| | 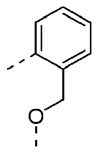 | (2-(1,3-dioxyisoindolin-2-yl)phenyl)methyl [1-(4-chlorobenzoyl)-5-methoxy-2-methyl-1*H*-indol-3-yl]acetate |
| 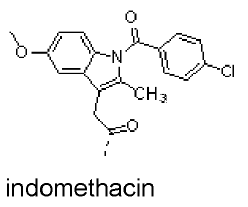 | | 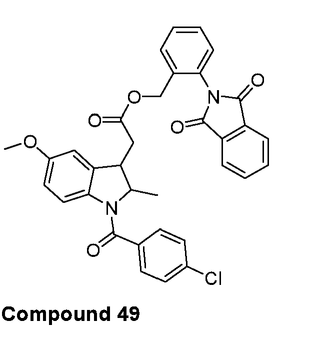 |
| | -NH- | 3,3'-(*E*)-diazene-bis-2-hidroxy-N-(1,3-dioxyisoindolin-2-yl)benzamide |
| 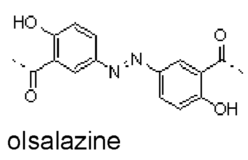 | | 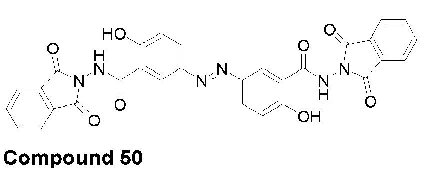 |
| | -CH₂O- | bis-(1,3-dioxy-1,3-dihydro-*2H*-isoindol-2-yl)methyl-3,3'-(*E*)-diazene-1,2-diylbis(6-hydroxybenzoate) |
| 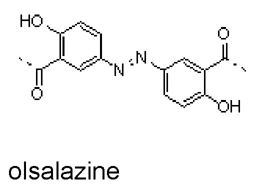 | | 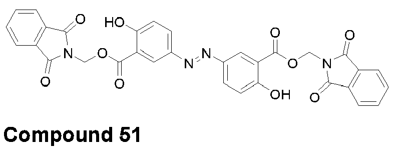 |
| | -CH₂CH₂O- | bis-(1,3-dioxy-1,3-dihydro-*2H*-isoindol-2-yl)ethyl-3,3'-(*E*)-diazene-1,2-diylbis(6-hydroxybenzoate) |
| 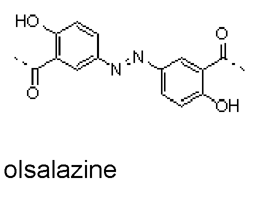 | | 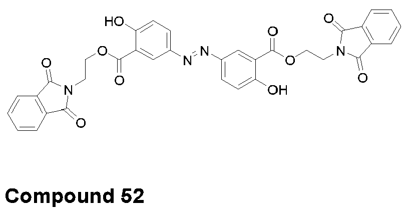 |
| | 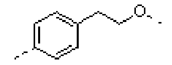 | bis-4-(1,3-dioxyisoindolin-2-yl)phenyl)ethyl-3,3'-(*E*)-diazene-1,2-diylbis(6-hydroxybenzoate) |
| 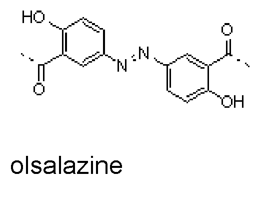 | | 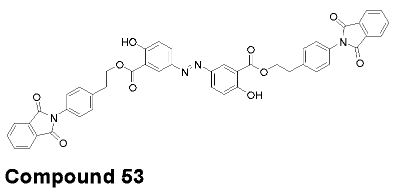 |
| | 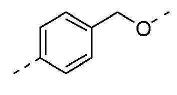 | bis-4-(1,3-dioxyisoindolin-2-yl)phenyl)methyl-3,3'-(*E*)-diazene-1,2-diylbis(6-hydroxybenzoate) |
| 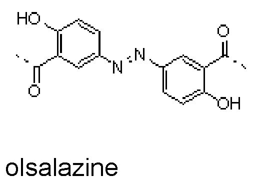 | | 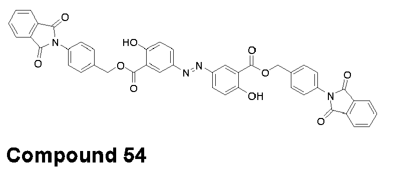 |
| | 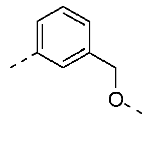 | bis-3-(1,3-dioxyisoindolin-2-yl)phenyl)methyl-3,3'-(*E*)-diazene-1,2-diylbis(6-hydroxybenzoate) |
| 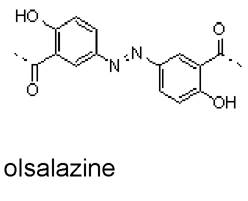 | | 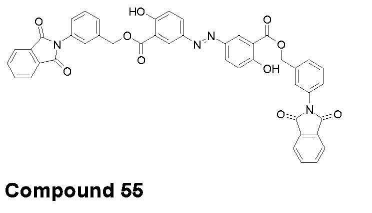 |
| | 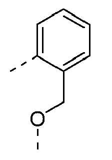 | bis-2-(1,3-dioxyisoindolin-2-yl)phenyl)methyl-3,3'-(*E*)-diazene-1,2-diylbis(6-hydroxybenzoate) |
| 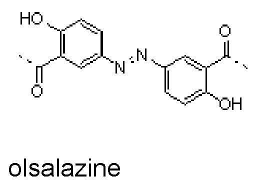 | | 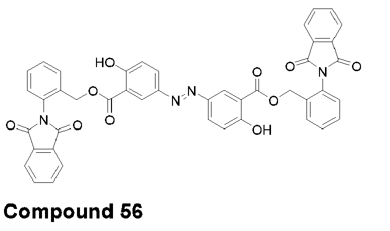 |
| | -NH- | 4-{(E)-[3-(hidrazynecarbonyl-N-(1,3-dioxyisoindolin-2-yl))-4-hydroxyphenyl]diazenyl}-N-piridin-2-ylbenzenesulfonamida |
| 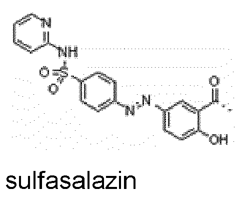 | | 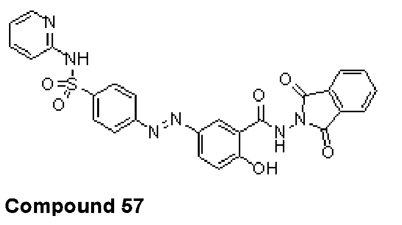 |
| | -CH₂O- | (1,3-dioxy-1,3-dihydro-*2H*-isoindol-2-yl)methyl 2-hydroxy-5-[(*E*)-{4-[(pyridin-2-ylamino)sulfonyl]phenyl}diazenyl]benzoate |
| 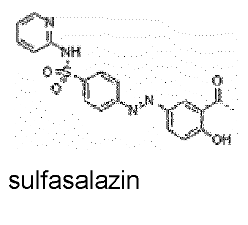 | | 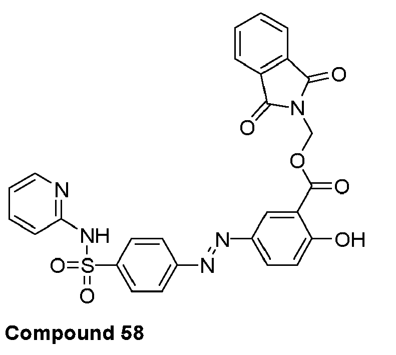 |
| | -CH₂CH₂O- | (1,3-dioxy-1,3-dihydro-*2H*-isoindol-2-yl)ethyl 2-hydroxy-5-[(E)-{4-[(pyridin-2-ylamino)sulfonyl]phenyl}diazenyl]benzoate |
| 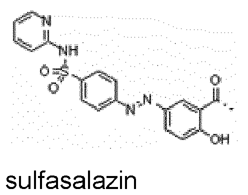 | | 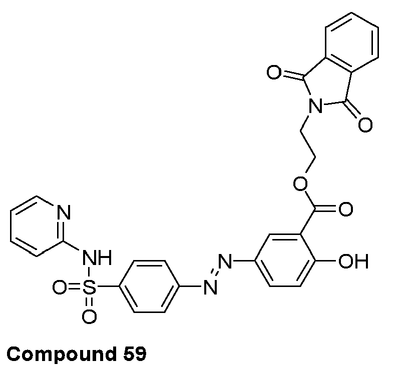 |
| | 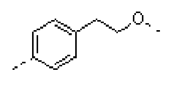 | (4-(1,3-dioxyisoindolin-2-yl)phenyl)ethyl 2-hydroxy-5-[(*E*)-{4-[(pyridin-2-ylamino)sulfonyl]phenyl}diazenyl]benzoate |
| 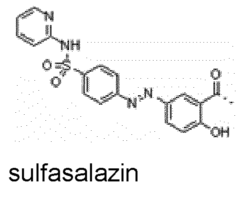 | | 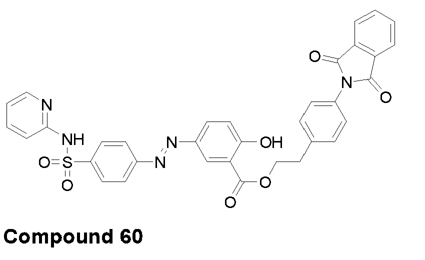 |
| | 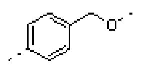 | 4-(1,3-dioxyisoindolin-2-yl)phenyl)methyl 2-hydroxy-5-[(*E*)-{4-[(pyridin-2-ylamino)sulfonyl]phenyl}diazenyl]benzoate |
| 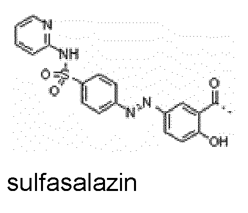 | | 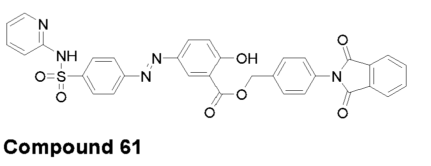 |
| | 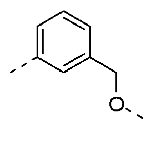 | 3-(1,3-dioxyisoindolin-2-yl)phenyl)methyl 2-hydroxy-5-[(*E*)-{4-[(pyridin-2-ylamino)sulfonyl]phenyl}diazenyl]benzoate |
| 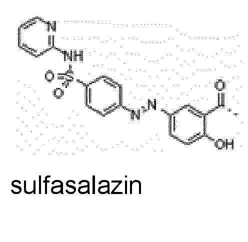 | | 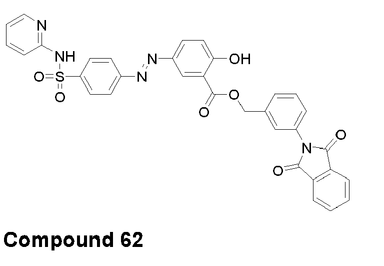 |
| | 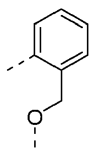 | 2-(1,3-dioxyisoindolin-2-yl)phenyl)methyl 2-hydroxy-5-[(*E*)-{4-[(pyridin-2-ylamino)sulfonyl]phenyl}diazenyl]benzoate |
| 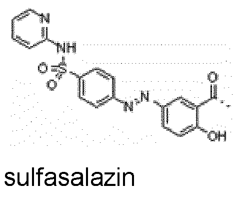 | | 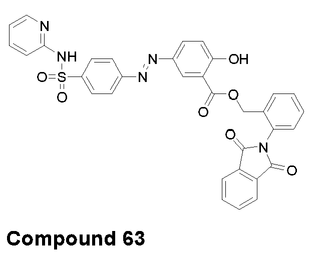 |

The process of the invention allows obtaining phthalimide derivatives from NSAIDs with surprisingly good performance. Scheme 1 illustrates the synthetic route of preparation of said derivatives of the invention, a route that is characterized by presenting a few synthetic stages, starting from the commercially available compounds, which qualifies this synthetic methodology for industrial use.

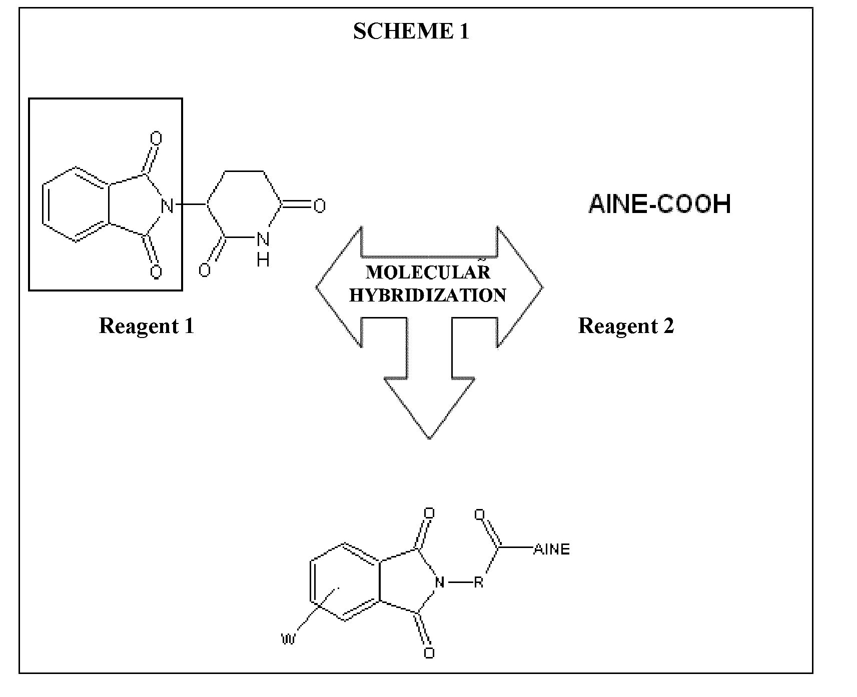

The phthalimide derivatives of this invention have been planned through convergent syntheses, using classic reactions such as: (i) Regioselective Nucleophilic Substitution; (ii) Nucleophilic Addition to Carbonyl, (iii) Condensation to form the Imide.

One of the synthesis routes illustrate the process of the invention is represented in Scheme 2 showing the synthesis of new phthalimide derivatives designed from the prototypes thalidomide (1) and nonsteroidal, anti-inflammatory compounds (NSAIDs) (2).

According to Scheme 2, the phthalimide derivatives of this invention are prepared by a process comprising the stages of: (a) esterification reaction by acid catalysis exploiting the nucleophilicity of methanol; (b) nucleophilic substitution by hydrazine, (c) condensation, under heating, of suitably functionalized amines using phthalic anhydride as the solvent, e.g. acetic acid or pyridine. In this process, heating can be replaced by microwave radiation, which enables reduction in reaction time.

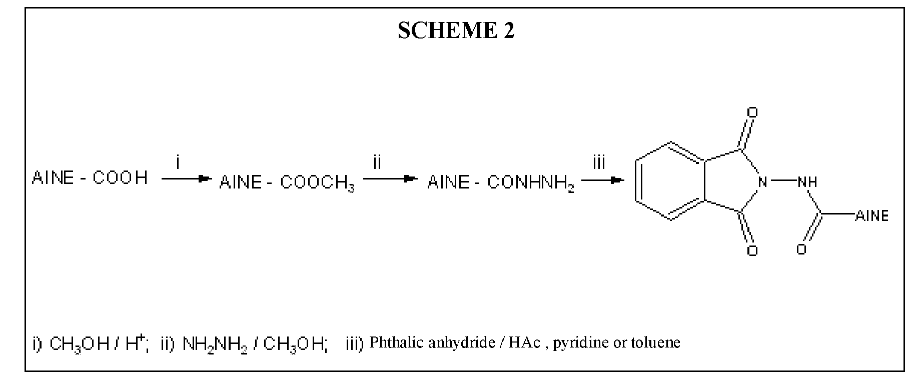

Alternatively, the phthalimide derivatives from NSAIDs of the invention can be prepared according to the synthetic route presented in Scheme 3.

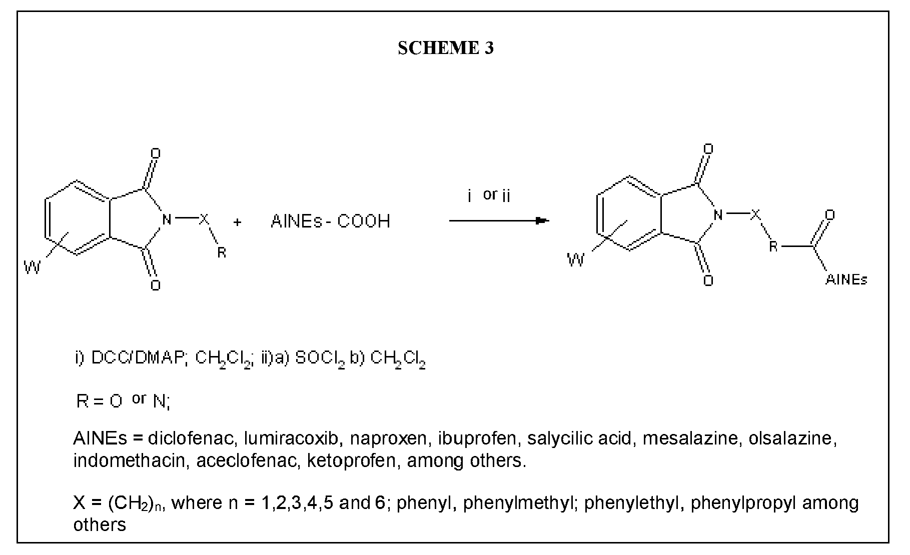

According to Scheme 3, the phthalimide derivatives of this invention are prepared by a process comprising the stages of: (a) Activation of the carboxylic acid of the anti-inflammatory agents using coupling agents (e.g. DCC) or halogenating agents (e.g. thionyl chloride); (b) Nucleophilic substitution of the activated carboxylic acid by phthalimide derivatives containing an amine or alcohol subunit to obtain hybrids containing functional groups of esters or amides.

The compounds of the invention can be administered in a variety of dosage forms; for example, orally as tablets, capsules, sugar or covered with film, liquid solutions or suspensions; rectally in the form of suppositories, parenterally, that is, intramuscular, or by infusion or intravenous and/or intratecal and/or intraspinal injection.

This invention also includes pharmaceutical compositions comprising phthalimide derivatives compounds, or pharmaceutically acceptable salts thereof, in association with a pharmaceutically acceptable excipient (which can be a carrier or diluent).

The pharmaceutical compositions containing the compounds of the invention are usually prepared following conventional methods and are administered in appropriate pharmaceutical form.

For instance, the solid oral pharmaceutical forms may contain, together with the active compound, diluents, lubricants, binding agents, disintegrating agents and others.

Examples of diluents that can be used in pharmaceutical compositions of the invention are: lactose, dextrose, sucrose, cellulose, starch or potato starch.

Examples of lubricants that can be used in pharmaceutical compositions of the invention are: silica, talc, stearic acid, magnesium or calcium stearate and/or polyethylene glycols.

Examples of binding agents that can be used in pharmaceutical compositions of the invention are: starches, gum Arabic, gelatin, methylcellulose, carboxymethylcellulose or polyvinyl pyrrolidone.

Examples of disintegrating agents that can be used in pharmaceutical compositions of the invention are: starch, alginic acid, alginates or starch or sodium glycolate, effervescent mixtures, dyes, sugar.

Additionally, they can be used in pharmaceutical compositions of the invention wetting agents such as lectin, polysorbates, lauryl sulfates and, in general, pharmacologically inactive substances and non-toxic commonly used substances in pharmaceutical formulations. The preparation of said pharmaceutical compositions of the invention can be implemented in a known fashion, for example, by mixing, granulation, pressing into tablet, sugar coating, or coating processes film.

The liquid dispersions for oral administration can be, for example, syrups, emulsions and suspensions. In addition to the active compound of this invention, the syrups may contain one or more carrier agents, for example, sucrose or sucrose with glycerin and/or mannitol and/or sorbitol. Suspensions and emulsions may contain as carrier, for example, a natural gum, agar, sodium alginate, pectin, methylcellulose, carboximethylcelulose or polyvinyl alcohol.

The suspensions or solutions for intramuscular injections may contain, together with the active compound of the invention, a pharmaceutically acceptable carrier, i.e. sterile water, olive oil, ethyl oleate, glycols such as propylene glycol, and if desired, the appropriate quantity of lidocaine hydrochloride. The solutions for intravenous injections or infusions may contain as carrier, for example, sterile water or preferably they may be in the form of sterile, aqueous, isotonic saline solutions or they may contain as carrier, propylene glycol.

The suppositories may contain together with the active compound of the invention, a pharmaceutically acceptable carrier, for example, cocoa butter, glycol polyethylene, sorbitol polyoxyethylene, fatty acid ester surfactant, or lecithin.

Below we present, by way of example, substantiations of the preparation of phthalimide derivatives from the NSAIDs of the invention. However, it should be understood that these examples are provided only for illustrative purposes and that various modifications or changes in light of the subsantiations disclosed here will be suggestive to experts in the technique and must be included within the spirit of this description and scope of the claims that are attached.

### EXAMPLES

### EXAMPLE 1

### Preparation of Compound N-(1,3-dioxy-1 ,3-dihydro-2H-isoindol-2-yl)-2-hydroxybenzamide

The synthesis of hybrid derivatives of salicylic acid of the invention can be represented schematically as follows: 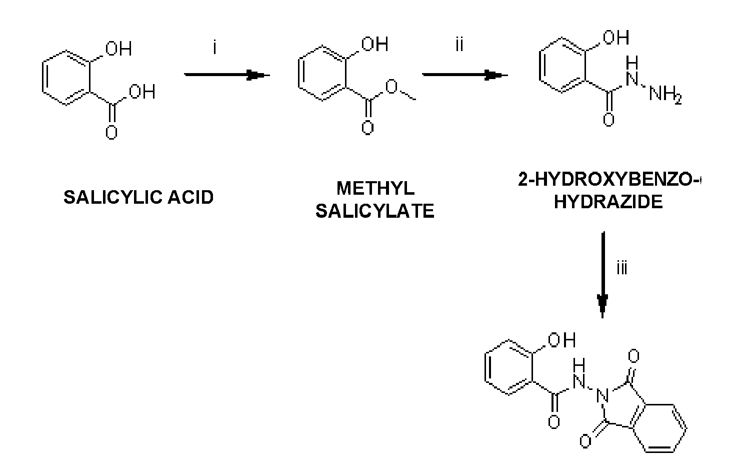 where the reaction conditions are as follows: i = methanol, reflux; ii = hydrazine hydrate 25%, methanol; iii = phthalic anhydride, acetic acid, reflux.

The following describes in detail the stages of the process to obtain phthalimide derivatives of salicylic acid.

### 1.1. Synthesis of methyl salicylate:

Methyl salicylate can be prepared according to known techniques or purchased commercially.

### 1.2. Synthesis of 2-hydroxybenzohydrazide intermediary

In a 125 mL flask, add 10 mL of methyl salicylate (77.5 mmol), 50 mL of methanol and 31 mL of hydrazine hydrate. The reaction is kept under reflux and stirring for 16 hours until thin layer chromatography indicates completion of the reaction (Mobile phase: 90% dichloromethane; 10% methanol).

The reaction is cooled and the solvent volume is decreased at reduced pressure to provide 10.62 g of a white solid with a melting range between 146° -149° C (C₇H₈O₂N; MW=152.14; yield: 90%).

The H¹ NMR spectrum (400MHz, DMSOd) is as follows: δ4.73 (s; 2H); 6.85-6.93 (2H); 7.38 (ddd; 1H; Jₒᵣₜₒ = 7.86 Hz and J_{Meta} =1.71 Hz); 7.83 (dd; 1H; Jₒᵣₜₒ = 7.94 Hz and Jₘₑₜₐ = 1.65 Hz); 10.09 (1H) ppm.

### 1.3. Synthesis of compound N (1,3-dioxy-1,3-dihydro-2H-isoindol-2-yl) - 2-hydroxybenzamide

In a 50 mL flask connected to a reflux condenser, add 0.5 g (3.28 mmol) of salicylic acid hydrazide obtained in stage 1.2, 15 mL of glacial acetic acid and 0.487 g of phthalic anhydride. The reaction is kept under reflux and stirring (130° C) for 3 hours until we can observe the formation of the product by thin layer chromatography (Mobile phase: 90% dichloromethane; 10% methanol).

The reaction is cooled in an ice bath, with the formation of precipitate which is filtered and washed with cold water to provide 787 mg of a white solid (C₁₅H₁₀N₂O₄, MW=282.06, melting point: 241°-244°C; yield: 85%).

The NMR H¹ spectrum (400MHz, DMSOd) is as follows: δ 6.99-7.08 (2H); 7.51 (ddd; 1H; Jₒᵣₜₒ=8.36 Hz and Jₘₑₜₐ=1.71 Hz ) 7.94 (dd; 1H; Jₒᵣₜₒ=7.85 Hz and Jₘₑₜₐ=1.71 Hz); 7.96 (m; 2H; Jₒᵣₜₒ=8.7 Hz and Jₘₑₜₐ=2.73 Hz); 8.02 (m; 2H; Jₒᵣₜₒ=8.7 Hz and Jₘₑₜₐ=2.73 Hz); 11.10 (1H) ppm.

The NMR C¹³ spectrum (400MHz, DMSOd) is as follows: δ 166.35; 165.47; 158.15; 135.6; 134.94; 130.14; 129.73; 124.03; 119.61; 117.46; 115.3 ppm.

### EXAMPLE 2

### Preparation of Compound N-(1,3-dioxy-1 ,3-dihydro-2H-isoindol-2-yl) -2 - (4-isobutylphenyl) propanamide

The synthesis of hybrid derivatives of isoprofen of the invention can be represented schematically as follows: 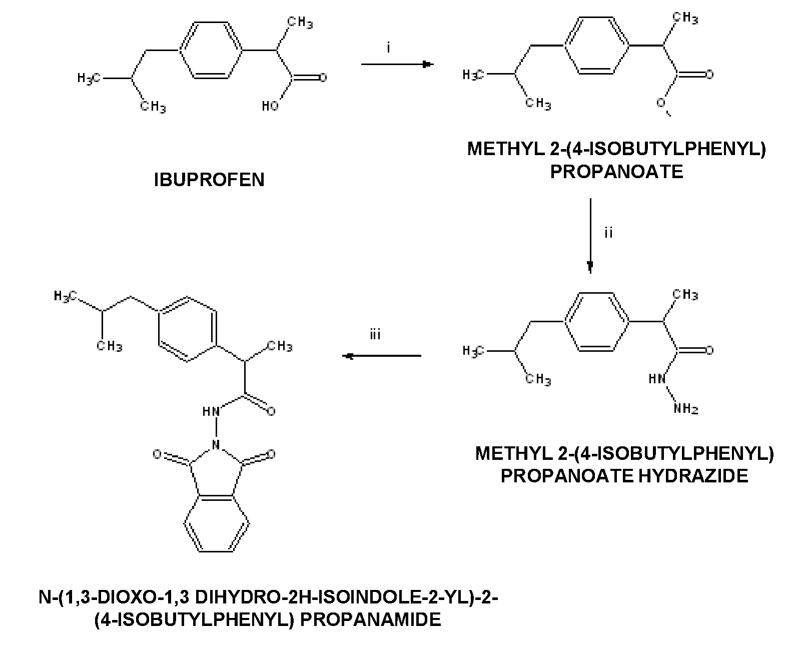 where the reaction conditions are as follows: i = methanol, ambient temperature; ii = hydrazine hydrate 25%; methanol; iii = phthalic anhydride, acetic acid, reflux.

### 2.1. Synthesis of the intermediary methyl 2- (4-isobutyl phenyl) propanoate

In a 125 mL flask were added 0.3 g (1.45 mmol) of ibuprofen, 20 mL of methanol (CH₃OH) and 2 drops of sulfuric acid (H₂SO₄). The mixture was kept under stirring at ambient temperature for 24 hours until thin layer chromatography indicated the completion of the reaction (Mobile Phase: 95% dichloromethane; 5% methanol).

The solvent was evaporated at room temperature at reduced pressure to obtain 0.32 g of a solid of white color with melting point (less than 70° C) (C₁₄H₂₀O2, MW= 220.3; yield: 100%).

The NMR H¹ spectrum (400MHz, DMSOd) is as follows: δ 0.83 (d; 6H); 1.36 (d; 3H); 1.79 (m; 1H); 2.39 (d; 2H); 3.55 (s; 3H); 3.73 (q; 1H); 7.09 (d; J= 8.02 Hz; 2H) and 7.16 (d; J= 8.02 Hz; 2H) ppm.

### 2.2. Synthesis of the intermediary 2-(4-isobutylphenyl) propanohydrazide

In a round-bottom shaped 125 mL flask connected to a reflux condenser were added 0.32 g of the ester of ibuprofen (1.45 mmol), 20 mL of methanol (CH₃OH) and 10 mL of hydrazine hydrate 25%.

The reaction was kept at 45°C-50°C for 24 hours until thin layer chromatography (Mobile phase: 90% dichloromethane, 10% methanol) could observe the completion of the reaction. When the reaction did not occur within the time described we added about 2 mL of hydrazine hydrate 25%, and followed the reaction for about 4 hours. The isolation of the reaction was done by reducing the solvent volume in about 12 mL, and then adding about 4 mL water/ice.

It was then observed the formation of a precipitate that was filtered and washed with 10 mL of ice water to provide 0.186 g of a white color solid at melting range between 78° - 82° C.

If the product does not precipitate with this process, sulfuric acid must be added until the pH is neutral; in this pH there is the formation of precipitate in the same way that the procedure described above; it can be filtered and washed with cold water (C₁₃H₂₀N₂O; MW = 220.3; yield: 58%).

The NMR H¹ spectrum (400MHz, DMSOd) is as follows: δ 0.83 (d; 6H); 1.30 (d; 3H); 1.78 (m; 1H); 2.38 (d; 2H); 3.47 (q; 1H); 4.20 (2H); 7.05 (d; J= 8.02 Hz; 2H) and 7.2-(d; J= 8.1 Hz; 2H); 9.14 (1H) ppm.

### 2.3. Synthesis of N-(1,3-dioxy-1,3-dihydro-2H-isoindol-2-yl)-2-(4-isobutylphenyl)propanamide

In a 50 mL flask connected to a reflux condenser there were added 0.32 g of ibuprofen ester (1.45 mmol), 0.215 g of phthalic anhydride and 7 mL of acetic acid. The reaction was kept under reflux and stirring for 5 hours until thin layer chromatography (Mobile phase: 95% dichloromethane, 5% methanol) could observe the completion of the reaction.

The reaction flask was cooled, and in ice bath it was added a solution of 20% sodium hydroxide until the pH was neutral. In pH 7 there occurred the formation of a precipitate that was filtered and washed with ice water to provide 0.4 g of a white solid at melting range between ° C (C₂₁H₂₂N₂O₃, MW= 350.41, yield: 78%).

The NMR H¹ spectrum (400MHz, DMSOd) was as follows: δ 0.83 (d; 6H); 1.39 (d; 3H); 1.81 (m; 1H); 2.41 (d; 2H); 3.64 (q; 1H); 7.11 (dd; J= 8.0 Hz; 2H); 7.19 (dd; J= 8.0 Hz; 2H) 7.89 (m; J= 9.1 Hz; 2H); 7.94 (s; 1H); 8.11 (m; 2H) ppm.

The NMR C¹³ spectrum (400MHz, DMSOd) was as follows: δ 173.22; 154.99; 139.84; 139.45; 138.11; 135.39; 132.7; 129.57; 129.08; 128.98; 127.42; 127.23; 127.17; 125.32; 123.8; 44.34; 29.71; 22.97; 18.93; 18.82; 18.47 ppm.

### EXAMPLE 3

### Preparation of Compound N-(1,3-dioxy-1 ,3-dihydro-2H-isoindol-2-yl) -2 - (6-methoxy-2-naftyl) propanamide

The synthesis of hybrid derivatives of naproxen of the invention can be represented schematically as follows: 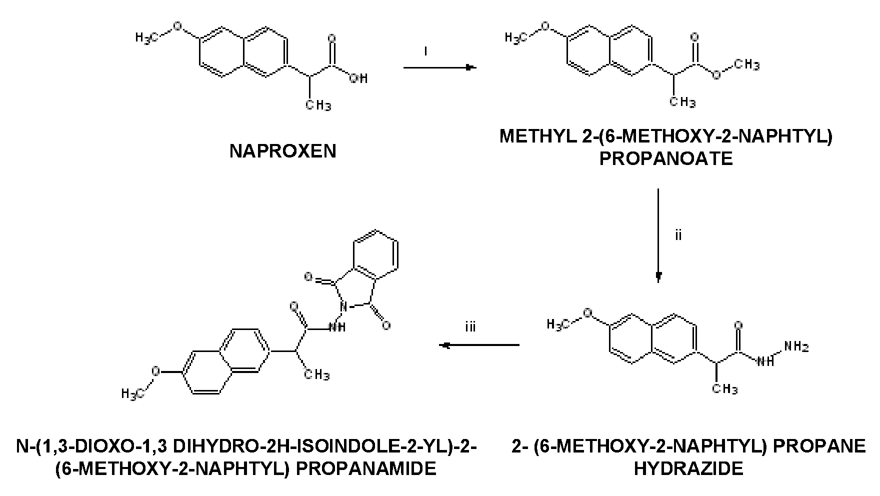 where the reaction conditions are as follows: i = methanol, reflux; ii = hydrazine hydrate 25%, methanol; iii = phthalic anhydride, acetic acid, reflux.

### 3.1. Synthesis of the intermediary methyl 2- (6-methoxy-2-naftyl) propanoate

In a 250 mL flask connected to a reflux condenser there were added 0.7 g (3 mmol) of naproxen, 50 mL of methanol and 2 drops of concentrated sulfuric acid. The reaction is kept under reflux and stirring for 6 hours until thin layer chromatography indicated the completion of the reaction (Mobile phase: 90% dichloromethane; 10% methanol).

The product was obtained by removing the solvent at reduced pressure to provide 0.74 g of an orange color solid with melting range between 88° - 94° C (C₁₅H₁₆O₃, MW=244.11, yield: 100%).

The NMR H¹ spectrum (400MHz, DMSOd) was as follows: δ 1.47 (d; 3H); 3.59 (s; 3H); 3.86 (s; 3H); 3.94 (q; 1H); 7.15 (dd; 1H; Jₒᵣₜₒ=8.7 Hz and Jₘₑₜₐ=2.56 Hz); 7.29 (d; 1H; Jₘₑₜₐ=2.56 Hz); 7.38 (dd; 1H; Jₒᵣₜₒ=8.53 Hz and Jₘₑₜₐ=1.87 Hz); 7.72 (d; 1H); 7.79 (Jₒᵣₜₒ= 8.87 Hz; 2H) ppm.

The IR spectrum (KBr pellet) was as follows: ν 2976 (C-H, CH₂ and CH₃) ν 1739.7 (C=O ₑₛₜₑᵣ), δ 1604, 1450 (C-H_{aromatic}); ν 1267 (C-O) cm⁻¹_{.}

### 3.2. Synthesis of the intermediary 2--(6-methoxy-2-naphtyl) propanohydrazide

In a 250 mL flask connected to a reflux condenser there were added 0.5 g of naproxen ester, 50 mL of methanol and 8 mL of hydrazine hydrate 25%. The reaction is kept under reflux and stirring for 24 hours until thin layer chromatography indicates the completion of the reaction (Mobile phase: 1:1, hexane:ethyl acetate).

The product was obtained by reduction of the solvent at reduced pressure, followed by addition of about 4 mL of water/ice. There is formation of a precipitate that is filtered and washed with ice water to provide 0.38 g of a white solid with melting range between 129° - 133° C. (C₁₄H₁₆N₂O₂ ; MW= 244,11; yield: 76%).

The NMR H¹ spectrum (400MHz, DMSOd) was as follows: δ 1.41 (d; 3H); 3.65 (q; 1H); 3.86 (s; 3H); 4.22 (s; 1H); 7.13 (dd; 1H; Jₒᵣₜₒ=9 Hz and Jₘₑₜₐ=2.56 Hz); 7.26 (d; 1H; Jₘₑₜₐ=2.39 Hz); 7.45 (dd; 1H; Jₒᵣₜₒ=8.53 Hz and Jₘₑₜₐ=1.88 Hz); 7.71 (d; 1H); 7.73 (d; 1H; Jₒᵣₜₒ= 8.53 Hz); 7.77 (d; 1H; Jₒᵣₜₒ= 9 Hz) ppm.

The IR spectrum (KBr pellet) was as follows: ν 3296 (N-H), 2956 (C-H, CH₂ and CH₃), ν 1637 (C=O _{hydrazide}), **δ** 1604, 1450 (C-H _{aromatic}), ν 1261 (C-O), ν 1213 (-CN) cm⁻¹.

### 3.3. Synthesis of N-(1,3-dioxy-1,3-dihydro-2H-isoindol-2-yl)-2-(6-methoxy-2-naphtyl)propanamide

In a 125 mL flask connected to a reflux condenser there were added 0.4 g (1.63 mmol) of naproxen hydrazide, 10 mL of glacial acetic acid and 0.243 g of phthalic anhydride (1.63 mmol). The reaction was kept under reflux (130° C) and magnetic stirring for about 3 hours until thin layer chromatography (Mobile phase: 1:1, hexane:ethyl acetate) could observe the reaction completion. After that time, the reaction mixture was cooled and it was added sodium hydroxide solution 20% until pH = 7.

There was formation of a precipitate that is filtered and washed with ice water to provide 0.509 g of a light yellow solid with melting range between °C (C₂₂H₁₈N₂O₄; MW = 374.13; yield: 83%).

The NMR H¹ spectrum (400MHz, DMSOd) was as follows: δ 1.53 (d; 3H); 3.88 (s; 3H); 4.06 (q; 1H); 7.18 (dd; 1H; Jₒᵣₜₒ=8.87 Hz and Jₘₑₜₐ=2.56 Hz); 7.32 (d; 1H; Jₘₑₜₐ=2.56 Hz); 7.52 (dd; 1H; Jₒᵣₜₒ=8.53 Hz and Jₘₑₜₐ=1.54 Hz); 7.82 (d; 3H); 7.94 (4H); 10.89 (s; 1H) ppm.

The NMR C¹³ spectrum (400MHz, DMSOd) was as follows: δ 173.03; 157.21; 135.9; 135.26; 133.37; 130.6; 129.48; 129.21; 128.41; 126.88; 126.36; 125.64; 123.7; 118.74; 105.76; 55.19; 42.95; 18.73 ppm.

The IR spectrum (KBr pellet) was as follows: ν 3276 ( N-H); ν 3030 (C-H _{aromatic}); 2956 (C-H CH₂ and CH₃); ν 1793 and 1741 ( C=O _{imide}); ν 1685 (C=O _{hydrazide}); δ 1606, 1467 (C-H _{aromatic}); ν 1263 (C-O); ν 1209 (C-N) cm⁻¹.

### EXAMPLE 4

### Preparation of Compound 2 - (3-benzoylphenyl)-N-(1,3-dioxy-1 ,3-dihydro-2H-isoindol-2-yl) propanamide

The synthesis of hybrid derivatives of ketoprofen of the invention can be represented schematically as follows: 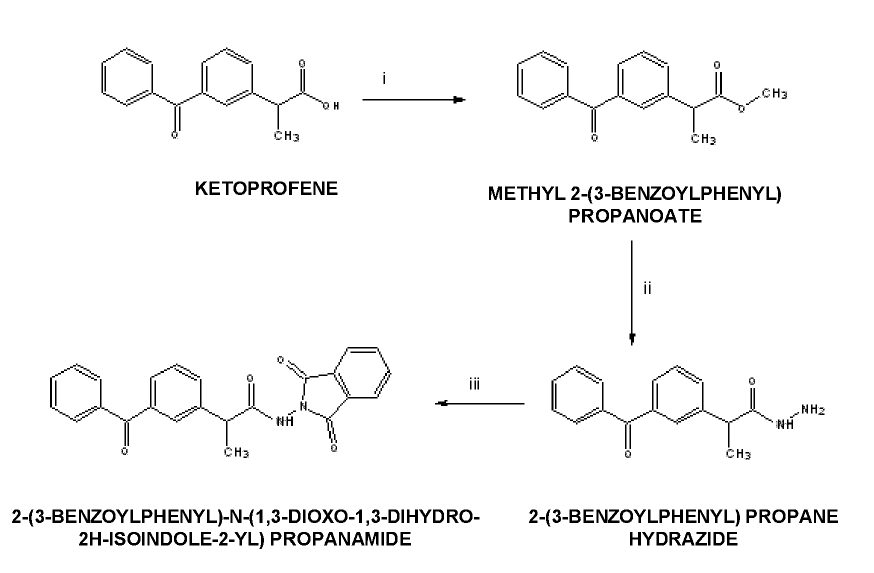 where the reaction conditions are as follows: i = methanol, reflux; ii = hydrazine hydrate 25%, methanol; iii = phthalic anhydride, acetic acid, reflux.

### 4.1. Synthesis of the intermediary methyl 2- (3-benzoylphenyl) propanoate

In a 250 mL flask connected to a reflux condenser there were added 0.5 g (1.96 mmol) of ketoprofen, 50 mL of methanol and 2 drops of concentrated sulfuric acid. The reaction is kept under heating at 50° C and stirring for 8 hours until reaching thin layer chromatography indicated the completion of the reaction (Mobile phase: 70% hexane; 30% ethyl acetate).

The product was obtained by removing the solvent at reduced pressure at room temperature to provide 0.527 g of a white color solid. (C₁₇H₁₆O₃; MW=268,11; yield: 100%).

The NMR H¹ spectrum (400MHz, DMSOd) was as follows: δ 1.48 (d; 3H); 3.65 (s; 3H); 3.98 (q; 1H); 7.52-7.59 (3H); 7.63-7.71 (3H); 7.76-7.81 (3H) ppm.

### 4.2. Synthesis of intermediary 2- (3-benzoylphenyl) propane-hydrazide

In a 250 mL flask connected to a reflux condenser there were added 0.5 g (1.86 mmol) ketoprofen ester, 50 of methanol and 9 mL of hydrazine hydrate 25%. The reaction was kept under heating and stirring for 24 hours until thin layer chromatography indicated the completion of the reaction (Mobile phase: 70% hexane: 30% ethyl acetate).

The product was obtained by the reduction of the solvent at reduced pressure, followed by addition of about 4 mL of water/ice. There is formation of a precipitate that is filtered and washed with cold water (c.a. 10 mL) to provide 0.375 g of a white color solid with melting range higher than 332° C (C₁₆H₁₆N₂O₂, MW=268.12, yield: 75%).

The NMR H¹ spectrum (400MHz, DMSOd) was as follows: δ 1.29 (d; 3H); 3.46 (q; 1H); 6.22 (s; 2H); 6.93 (dd; 1H; Jₒᵣₜₒ= 8.1 Hz); 7.14-7.22 (4H); 7.56-7.58 (3H); 9.16 (s; 1H) ppm.

### 4.3. Synthesis of the 2- (3-benzoylphenyl) - N (1,3-dioxy-1,3-dihydro-2H-isoindol-2-yl) propanamide

In a 125 mL flask connected to a reflux condenser there were added 0.5 g (1.86 mmol) of ketoprofen hydrazide, 10 mL of glacial acetic acid and 0.277 g of phthalic anhydride (1.86 mmol).

The reaction was kept under reflux (130° C) and magnetic stirring for about 5 hours until thin layer chromatography could observe the reaction completion. After that time, the reaction mixture was cooled and it was added sodium hydroxide solution 20% until pH = 7.

There was formation of a precipitate that was filtered and washed with ice water to provide 0.505 g of a white solid with melting range between ° C (C₂₄H₁₈N₂O₄; MW = 542.52; yield: 68%).

The NMR H¹ spectrum (400MHz, DMSOd) was as follows: δ 1.45 (d; 3H); 4.01 (s; 1H); 7.58 (dd; 1H; Jₒᵣₜₒ= 8.1 Hz); 7.66 (dd) ; 7.78 (dd) ; 7.94 (m); 8.13 (m; 2H); 8.07 (m; 2H) 9.16 (s; 1H) ppm.

The IR spectrum (KBr pellet) was as follows: ν 3276 (N-H); ν 3030 (C-H _{aromatic}); 2956 (C-H CH₂ and CH₃); ν 1793 and 1741 (C=O _{imide}); ν 1685 (C=O _{hydrazide}); δ1606, 1467 (C-H _{aromatic}); δ 1263 (C-O); ν 1209 (C-N) cm⁻¹.

### EXAMPLE 5

### Preparation of Compound 2-{2-[(2,6-dichlorophenyl)amine]phenyl} -N-(1,3-dioxy-1,3-dihydro-2H-isoindol-2-yl)acetamide

The synthesis of hybrid derivatives of diclofenac of the invention can be represented schematically as follows: 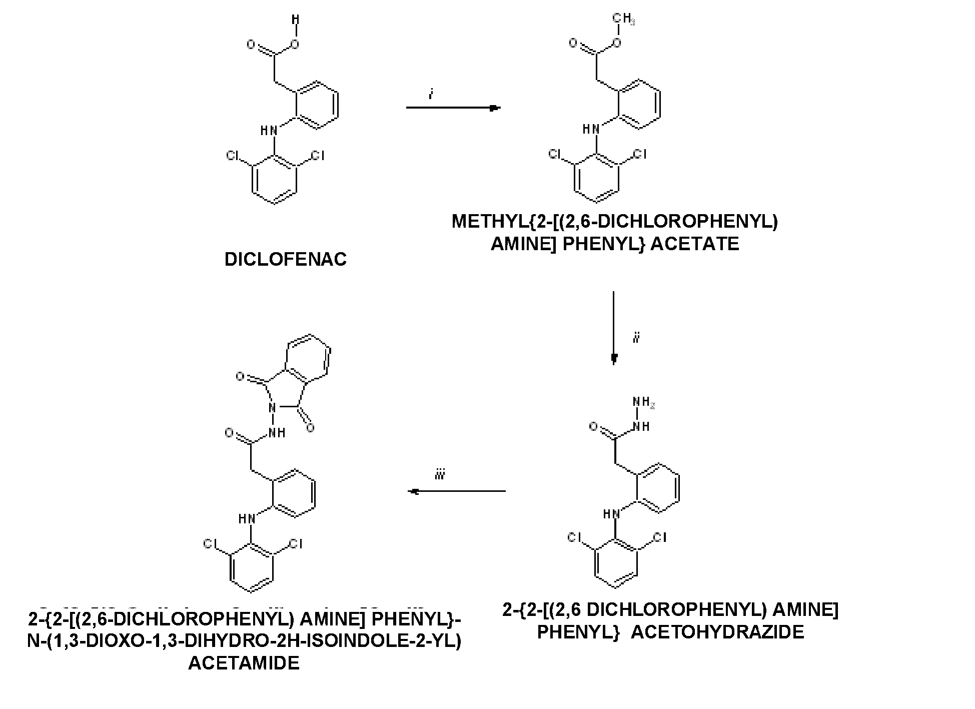 where the reaction conditions are as follows: i = methanol, reflux; ii = hydrazine hydrate 25%, methanol; iii = phthalic anhydride, acetic acid, reflux.

### 5.1. Synthesis of the intermediary methyl {2- [(2,6-dichlorophenyl) amine] phenyl} acetate

In a 125 mL flask were added 500 mg (1.68 mmol) of diclofenac, 40 mL of methanol and 3 drops of concentrated hydrochloric acid. The reaction is kept under agitation and reflux for 6 hours until reaching the reaction monitoring by thin layer chromatography ( TLC) indicated the end of the reaction (Mobile Phase: 90%CH₂Cl₂: 10% MeOH). The reaction volume was reduced to ~15 mL, ice was added to the reaction mixture (~ 4 mL) and the pH was neutralized using solution of saturated sodium bicarbonate. There was the formation of a white precipitate which is filtered and washed with cold water to provide 480 mg (91%) of the methyl ester defined in title (melting range= 279° C - 282° C; MW = 310.175; C₁₅H₁₃C1₂NO₂) .

### 5.2. Synthesis of intermediary 2- {2- [(2,6-dichlorophenyl) amino] phenyl} acetohydrazide

In a 125 mL flask were added 500 mg (1.61 mmol) of methyl ester of diclofenac obtained in the previous stage, 20 mL of methanol and 6 mL of hydrazine hydrate 25%. The reaction was kept under agitation and reflux for 24 hours until the reaction monitoring by thin layer chromatography ( TLC) indicated the end of the reaction (Mobile Phase: 90%CH₂Cl₂: 10% MeOH). The reaction volume was reduced to ~ 8 mL, ice was added to the reaction mixture (~ 4 mL). The mixture was stored overnight in the refrigerator. There was the formation of a white precipitate which was filtered and washed with cold water to provide 450 mg (90%) of the diclofenac hydrazide (melting range= 160° C - 161.6° C; MW = 310.175; C₁₅H₁₃Cl₂N₃O).

### 5.3. Synthesis of the 2-{2-[(2,6-dichlorophenyl)amino]phenyl}-N-(1,3-dioxy-1,3-dihydro-2H-isoindol-2-yl)acetamide

In a 50 mL flask were added 100 mg of diclofenac hydrazide obtained in the previous step, 45 mg of phthalic anhydride and 7 ml of glacial acetic acid. The reaction was kept under stirring and reflux for 4 hours until the reaction monitoring by thin layer chromatography ( TLC) indicated the end of the reaction (Mobile Phase: 90%CH₂Cl₂: 10% MeOH). The reaction mixture is cooled and there was precipitation of a white solid, which was filtered and washed with cold water to provide 130 mg (96%) of the final product (melting range = 255.8° C- 259.6° C, MW = 440.28; C₂₂H₁₅Cl₂N₃O₃).

The IR spectrum (KBr pellet) was as follows: v 3276 ( N-H); ν 3030 (C-H aromatic); 2956 (C-H CH₂ and CH₃); ν 1793 and 1741 ( C=O imide); ν 1685 (C=O hydrazide); δ1606, 1467 (C-H aromatic); ν 1263 (C-O); ν 1209 (C-N) cm⁻¹

The NMR H¹ spectrum (400MHz, DMSOd) was as follows: δ 11.1 (N-H; 1H); 7.95 (m; 4H); 7.52 (dd; 1H); 7.50 (dd; 1H); 7.46 (d; 1H); 7.31 (N-H; d); 7.17 (ddd; 1H); 7.08 (ddd; 1H); 6.91 (ddd; 1H); 3.82 (s; 2H) ppm.

The NMR C¹³ spectrum (400MHz, DMSOd) was as follows: δ 170.68; 164.81; 142.77; 136.96; 135.20; 129.37; 129.06; 123.84; 123.70; 120.79; 115.99; 36.39 ppm.

### EXAMPLE 6

### Preparation of Compound 2-{2-[(2-chloro-6-fluorophenyl)amine]-5-methylphenyl}-N-(1,3-dioxy-1,3-dihydro-2H-isoindol-2-yl)acetamide

The synthesis of hybrid derivatives of lumiracoxib of the invention can be represented schematically as follows: 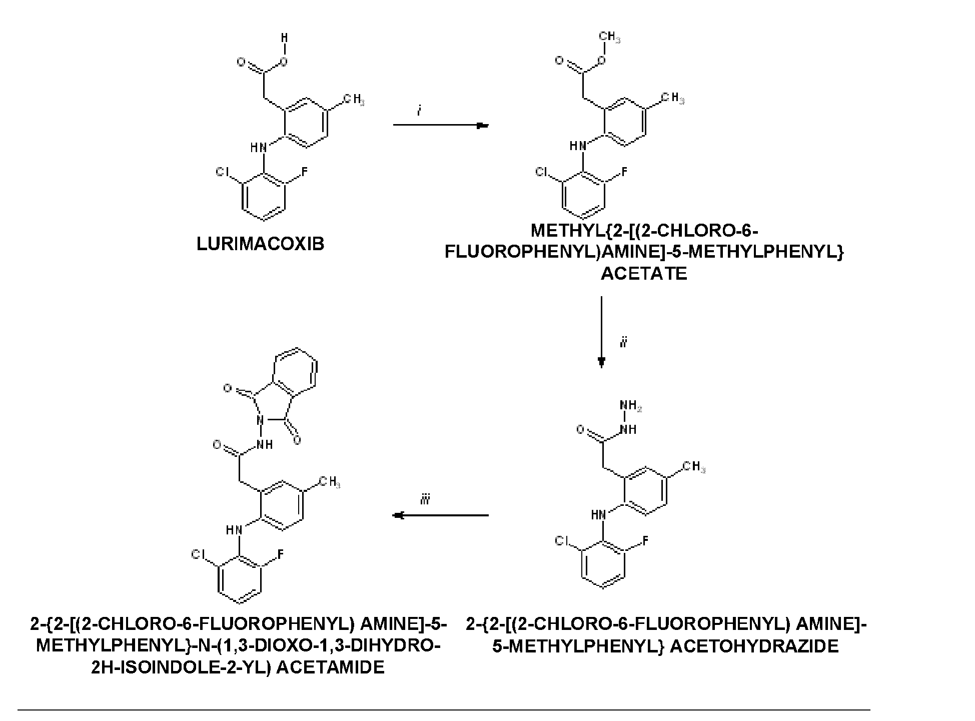 where the reaction conditions are as follows: i = methanol, reflux; ii = hydrazine hydrate 25%, methanol; iii = phthalic anhydride, acetic acid, reflux.

### 6.1. Synthesis of the intermediary methyl {2- [(2-chloro-6-fluorophenyl) amine] - 5-methylphenyl} acetate

In a 125 mL flask were added 500 mg (1.68 mmol) of lumiracoxib, 40 mL of methanol and 3 drops of concentrated hydrochloric acid. The reaction was kept under stirring and reflux for 8 hours until the reaction monitoring by thin layer chromatography ( TLC) indicated the end of the reaction (Mobile Phase: 90%CH₂Cl₂: 10% MeOH). The reaction volume was reduced to ~15 mL, ice was added to the reaction mixture (~ 4 mL) and the pH was neutralized using solution of saturated sodium bicarbonate. There was the formation of a white precipitate which was filtered and washed with cold water to provide 470 mg (89%) of the methyl ester. (Melting range= 136° C - 140° C MW = 307.74; C₁₆H₁₅C₁FNO₂).

### 6.2. Synthesis of intermediary 2-{2-[(2-chloro-6-fluorophenyl)amine]-5-methylphenyl}aceto-hydrazide

In a 125 mL flask were added 500 mg (1.62 mmol) of methyl ester of lumiracoxib, 20 mL of methanol and 6 mL of hydrazine hydrate 25%. The reaction was kept under stirring and reflux for 24 hours until the reaction monitoring by thin layer chromatography ( TLC) indicated the end of the reaction (Mobile Phase: 90%CH₂Cl₂: 10% MeOH). The reaction volume was reduced to ~ 8 mL, ice was added to the reaction mixture (~ 4 mL). The mixture was stored overnight in the refrigerator. There was the formation of a white precipitate which was filtered and washed with cold water to provide 400 mg (80%) of the lumiracoxib hydrazide. (Melting range= 166.4° C - 171°C MW = 307.74; C₁₆H₁₅C₁FNO₂).

### 6.3. Synthesis of 2-{2-[(2-chloro-6-fluorophenyl)amine]-5-methylphenyl}-N-(1,3-dioxy-1,3-dihydro-2H-isoindol-2-yl)acetamide

In a 50 mL flask it was added 190 mg of lumiracoxib hydrazide, 91,5 mg of phthalic anhydride and 10 ml of glacial acetic acid. The reaction was kept under stirring and reflux for 7 hours until the reaction monitoring by thin layer chromatography ( TLC) indicated the end of the reaction (Mobile Phase: 90%CH₂Cl₂: 10% MeOH). The reaction mixture is cooled and there was precipitation of a white solid, which was filtered and washed with cold water to provide 200 mg (74%) of the final product (melting range = 104.2° C- 109.1° C, MW = 437.85; C₂₃H₁₇ClFN₃O₃).

The IR spectrum (KBr pellet) was as follows: ν 3282 ( N-H); ν 3025 (C-H ₐᵣₒₘₐₜᵢc); 2917 (C-H CH₂ and CH₃); ν 1789 and 1737 ( C=O _{imide}); ν 1677 (C=O _{hydradize}); δ1603, 1454 (C-H _{aromatic}); ν 1271 (C-O); ν 1215 (C-N) cm⁻¹

The NMR H¹ spectrum (400MHz, DMSOd) was as follows: δ 7.95 (m; 2H); 7.61 (dd; 1H); 7.37 (m; 2H); 7.12 (dd; 1H); 7.07 (N-H; s; 1H); 7.01 (d; 1H); 6.71 (dd; 1H); 6.54 (dd; 1H); 3.85 (s; 2H); 2.31 (s; 3H) ppm.

The NMR C¹³ spectrum (400MHz, DMSOd) was as follows: δ 170.68; 163.6; 156.21; 150.17; 149.7; 133.29; 133.09; 132.6; 131.16; 130.85; 128.94; 126.85; 125.64; 125.07; 123.85; 119.31; 116.8; 166.1; 114.6; 107.01; 36.36; 21.05 ppm.

### EXAMPLE 7

This summary refers to the hybrid (reciprocal) of derivatives of diclofenac as per schemes relating to items 7.1 and 7.2.

### 7.1. Synthesis of 2-(1,3-dioxy-1,3-dihydro-2H-isoindol-2-yl)ethyl-{2-[(2,6-dichlorophenyl)amino]phenyl} acetate

Obtaining the title compound takes place as per the following scheme:

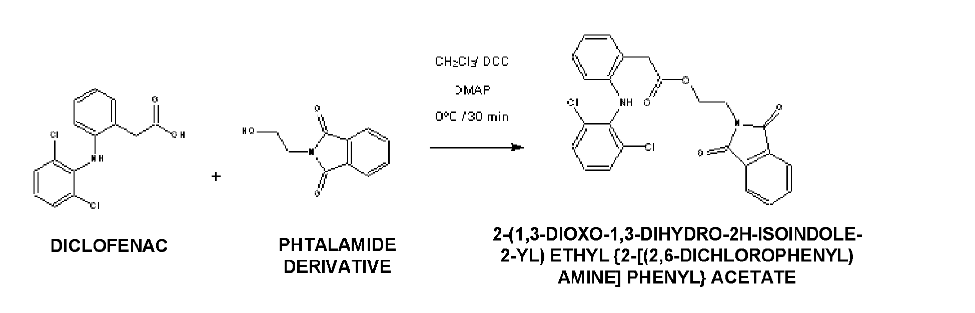

In a 25 mL flask there were added 0.2 g of diclofenac (0.677 mmol), 0.130 g (0.677 mmol) of phthalmidic derivatives, 0.0083 g (0.0677 mmol) of dimethylaminopyridine (DMAP) and 0.28 g (1.35 mmol) of diclohexylcarbohyimide (DCC) in 10 mL of distilled dichloromethane. The reaction was kept under vigorous stirring at 0°C until the reaction monitoring by thin layer chromatography (TLC) indicated the end of the reaction (Mobile Phase: 50% hexane; 50% ethyl acetate). The reaction mixture was filtered and the solid obtained was washed with 25 mL of distilled dichloromethane. The filtrate was evaporated at reduced pressure to obtain a white solid that was subsequently purified by chromatography column (silica; Mobile Phase: 80% hexane, 20% of ethyl acetate) to provide 150 mg (50%) of beige solid (melting range= 100.5° C - 104° C, MW = 469.3; C₂₄H₁₈Cl₂N₂O₄).

The IR spectrum (KBr pellet) was as follows: ν 3336 ( N-H); ν 3067 (C-H aromatic); 2917 (C-H CH₂); ν 1773 and 1716 ( C=O imide); δ51576, 1565, 1451 (C-H aromatic) ; ν 1270 (C-O); V 1213 (C-N) cm⁻¹.

The NMR H¹ spectrum (400MHz, DMSOd) was as follows: δ 7.8 (m; 4H); 7.45 (dd; 2H; Jo=8.05 Hz); 7.17 (dd; 1H); 7.09 (dd; 1H); 6.99 (ddd; 1H; Jₒ=7.7 Hz e Jₘ 1.6Hz); 6.83 (N-H; s; 1H); 6.75 (ddd; 1H; Jₒ= 7.6 Hz); 6.17 (dd; 1H; Jₒ=8.05 Hz); 4.31 (t; 2H); 3.86 (t; 2H); 3.72 (s; 2H) ppm.

The NMR C¹³ spectrum (400MHz, DMSOd) was as follows: δ 171.31;167.73; 142.74; 136.96; 134.34; 131.41; 130.89; 130.65; 129.054; 127.68; 125.87; 123.02; 122.81; 120.55; 115.73; 61.63; 36.84; 36.65 ppm.

MS-IE: 471 (m/z).

### 7.2. Synthesis of (1,3-dioxy-1,3-dihydro-2H-isoindol-2-yl)methyl {2-[(2,6-dichlorophenyl)amino]phenyl}acetate

Obtaining the title compound takes place as per the following scheme:

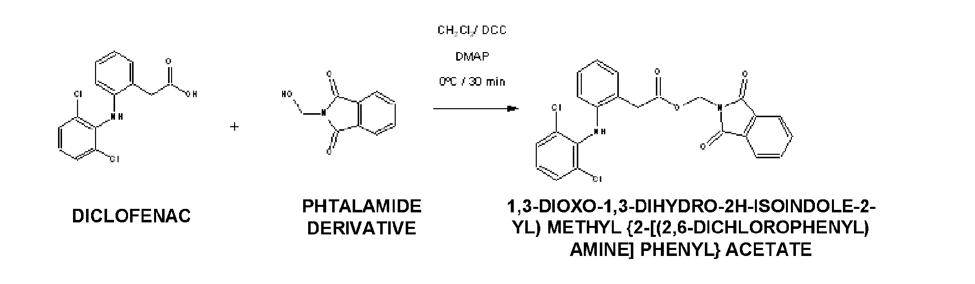

In a 25 mL flask there were added 0.2 g of diclofenac (0.677 mmol), 0.130 g (0.677 mmol) of phthalmidic derivatives, 0.0083 g (0.0677 mmol) of of dimethylaminopyridine (DMAP) and 0.28 g (1.35 mmol) of diclohexylcarbohyimide (DCC) in 10 mL of distilled dichloromethane. The reaction was kept under vigorous stirring at 0°C until the reaction monitoring by thin layer chromatography (TLC) indicated the end of the reaction (Mobile Phase: 50% hexane : 50% ethyl acetate). The reaction mixture was filtered and the solid obtained is washed with 25 mL of distilled dichloromethane. The filtrate was evaporated at reduced pressure to obtain a white solid that was subsequently purified by chromatography column (silica; Mobile Phase: 80% hexane, 20% of ethyl acetate) to provide 158 mg (52%) of beige solid (melting range= 153° C - 159° C; MW = 455.3; C₂₃H₁₆Cl₂N₂O₄).

The IR spectrum (KBr pellet) was as follows: ν 3388 ( N-H); ν 3028 (C-H aromatic); 2922 (C-H CH₂); ν1778 and 1729 ( C=O imide); δ 1590, 1561, 1451 (C-H aromatic); V 1274 (C-O); V 1211 (C-N) cm⁻¹.

The NMR H¹ spectrum (400MHz, DMSOd) was as follows: δ 7.9 (m; 4H); 7.45 (dd; 2H; Jo=8.05 Hz); 7.17 (dd; 1H); 7.09 (dd; 1H); 6.99 (ddd; 1H; Jₒ=7.7 Hz and Jₘ= 1.6Hz;); 6.83 (N-H; s; 1H); 6.75 (ddd; 1H; Jₒ= 7.6 Hz); 6.17 (dd; 1H; Jₒ=8.05 Hz); 5.6 (s; 2H); 3.72 (s; 2H) ppm.

The NMR C¹³ spectrum (400MHz, DMSOd) was as follows: δ 171.31;167.73; 142.74; 136.96; 134.34; 131.41; 130.89; 130.65; 129.054; 127.68; 125.87; 123.02; 122.81; 120.55; 115.73; 61.63; 36. 5 ppm.

MS-IE: 406 (m/z).

### TESTS OF THE RAT PAW EDEMA INCUDED BY CARRAGEENIN UTILIZING THE COMPOUNDS OF THE INVENTION OBTAINED IN EXAMPLES 1.3, 2.3, 3.3, 5.3 and 6.3

The anti-inflammatory activity of new phthalmidic derivatives claimed in this patent was evaluated through the model of paw edema induced by carrageenin. In this model all the new anti-inflammatory derivatives, tested at a concentration of 100 µmol/kg, showed anti-inflammatory activity, sometimes higher than standard NSAIDs.

Moreover, it is important to note that, according to the structural planning, new derivatives must have the ability to inhibit pro-inflammatory cytokine TNF-α which is present in large concentrations in chronic inflammatory processes; for example, rheumatoid arthritis and Chron's disease.

### EXAMPLE 8

### Test of Paw Edema Induced by Carrageenin with pre-treatment with the compound N-(1,3-dioxy-1 ,3-dihydro-2H-isoindol-2-yl)-2-hydroxybenzamide (derivative from salicylic acid)

The anti-inflammatory evaluation of the compound prepared according to Example 1.3 was performed through the model of rat paw edema induced by carrageenan administered orally in a single dose of 100 µmol/kg.

As shown in Figure 1, the compound of Example 1.3 showed significant anti-inflammatory activity compared to positive control (carrageenan), demonstrating the anti-inflammatory profile of said compound.

### EXAMPLE 9

### Test of Paw Edema Induced by Carrageenin with pre-treatment with the compound N-(1,3-dioxy-1,3-dihydro-2H-isoindol-2-yl)-2-(4-isobutylphenl) propanamide

The anti-inflammatory evaluation of the compound prepared according to Example 2.3 was performed through the model of rat paw edema induced by carrageenan administered orally in a single dose of 100 µmol/kg.

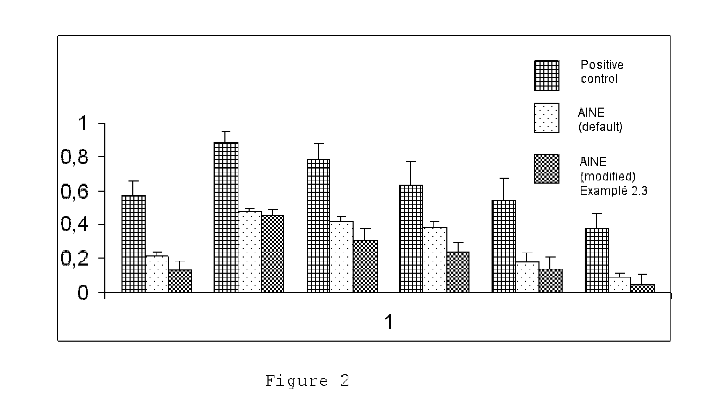

As shown in Figure 2, the compound of Example 2.3 showed significant anti-inflammatory activity compared to positive control (carrageenan), and superior activity compared with ibuprofen, NSAID used as standard on this test.

### EXAMPLE 10

### Test of Paw Edema Induced by Carrageenin with pre-treatment with the compound N-(1,3-dioxy-1,3-dihydro-2H-isoindol-2-yl)-2-(6-methoxy-2-naphtyl) propanamide

The anti-inflammatory evaluation of the compound prepared according to Example 3.3 was performed through the model of rat paw edema induced by carrageenan administered orally in a single dose of 100 µmol/kg.

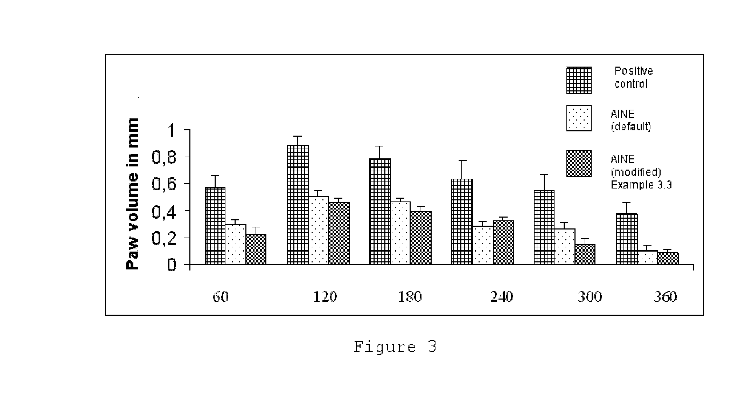

As shown in Figure 3, the compound of Example 3.3 showed significant anti-inflammatory activity compared to positive control (carrageenan), and superior activity compared with ibuprofen, NSAID used as standard on this test.

It is important to note that during the first hours of the inflammatory process there is effective participation of pro-inflammatory cytokine TNF-α. This cytokine has higher concentrations in the course of inflammation, i.e., in times greater than 360 minutes used in the test. This means that one is not evaluating the contribution of the inhibiting phthalmidic subunit of the synthesis of TNF-α. Therefore, although the anti-inflammatory activity at the first moment has been much greater than naproxen, the compound presents a different anti-inflammatory profile, in addition to greater safety, which justifies its use instead of naproxen which is available in drug therapy.

### EXAMPLE 11

### Test of Paw Edema induced by carrageenin with pre-treatment with the compound 2 - {2 - [(2,6-dichlorophenyl) amine] phenyl}-N-(1,3-dioxy-1 ,3-dihydro-2H-isoindole-2-yl) acetamide

The anti-inflammatory evaluation of the compound prepared according to Example 5.3 was performed through the model of rat paw edema induced by carrageenan administered orally in a single dose of 100 µmol/kg.

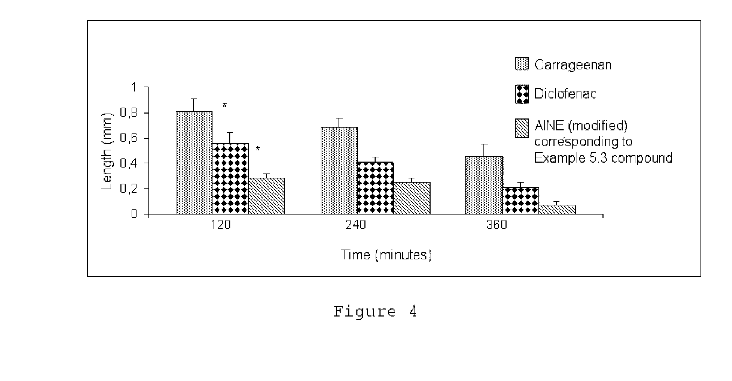

As shown in Figure 4, the compound of Example 5.3 showed significant anti-inflammatory activity when compared to positive control (carrageenan), and to diclofenac, used as standard NSAID in this test.

During the time of 120 minutes it was observed a significantly higher anti-inflammatory activity than that of diclofenac, indicating that the compound of Example 5.3, designed as anti-inflammatory, can be used in the acute inflammation phase. This same activity profile is kept at times 240 and 360 minutes. Example 5.3 can be characterized as a potent anti-inflammatory agent as well as more effective and safer than diclofenac, which is the drug available for treatment, and may even be a new alternative to the treatment of acute and chronic inflammatory processes.

### EXAMPLE 12

### Test of Paw Edema induced by carrageenin with pre-treatment with the compound 2-{2-[(2-chloro-6-fluorophenyl)amino]-5-methylphenyl}-N-(1,3-dioxy-1,3-dihydro-2H-isoindol-2-yl)acetamide

The anti-inflammatory evaluation of the compound prepared according to Example 6.3 was performed through the model of rat paw edema induced by carrageenan administered orally in a single dose of 100 µmol/kg.

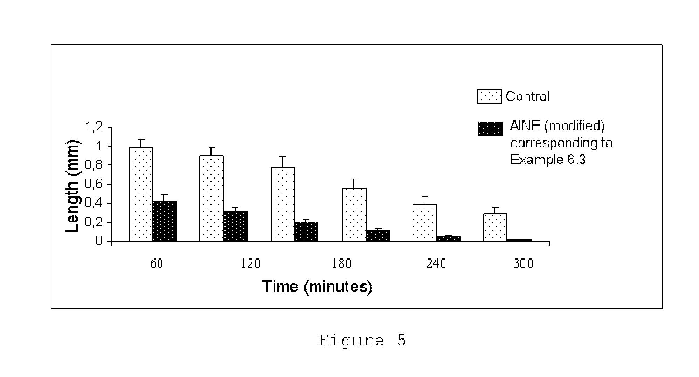

As shown in Figure 5, the compound of Example 6.3 showed significant anti-inflammatory activity when compared to positive control (carrageenan), demonstrating the anti-inflammatory profile of said compound.

### EXAMPLE 13

### Tests of gastric damage using the compounds of the invention obtained in examples 1.3, 2.3, 3.3, 5.3 and 6.3

We performed the evaluation of gastric lesions caused by new anti-inflammatory derived from phthalimide of the invention and compared with standard anti-inflammatory drugs (without molecular modification). The compounds were classified according to the grade of damage caused on a scale ranging from 1-5. It was assumed that: lesions <1 mm = grade 1, 1.5 to 2.5 mm = grade 2, 2.5 to 3.5 mm = grade 3, 3.5 to 4.5 mm, and grade 4 => 4.5 mm = grade 5. The results obtained were reported as averages ± E. P. M. All results were submitted to the variance homogeneity test (Levene's Test) to confirm homogeneity. The results with not significant p (above 0.05), were subsequently subjected to Variance Analysis (ANOVA) followed by multiple comparison test (post hoc analyses) such as the Newman - Keul's test, and only the values of p were considered when they were equal or below 0.05.

It was considered ground zero for the ulceration of compounds that do not damage the gastric lining. The stomachs were also evaluated on the number of lesions observed.

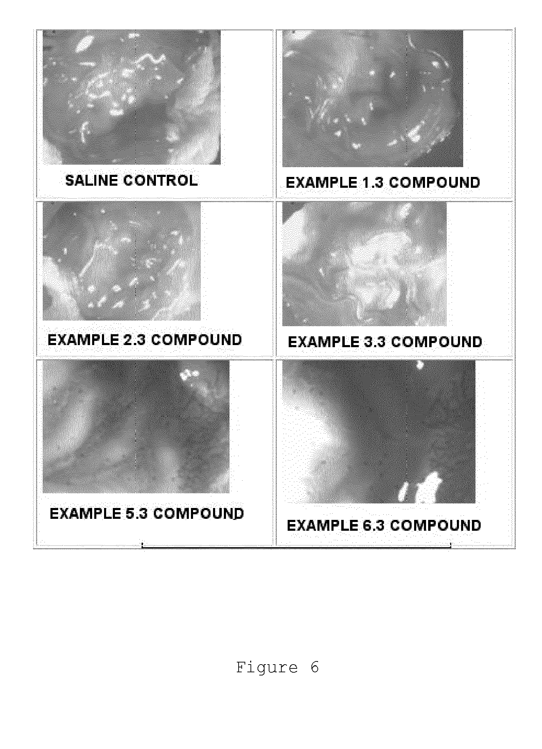

Figure 6 shows pictures of the stomachs of rats treated with phthalimide derivatives administered only once at the dosage of 100 µmol/kg and saline showing the absence of gastric ulceration.

### Treatment with the compound N-(1,3-dioxy-1 ,3-dihydro-2H-isoindol-2-yl)-2-hydroxybenzamide (Example 1.3)

The total area of lesions was of 2.9 mm² observed in six stomachs, a much lower number when compared with salicylic acid, which is the NSAID used as default. Moreover, as to the grade of ulceration we can classified the salicylic acid as grade 5, while the new compound can be classified as grade 1. This confirms that molecular modification used markedly reduced the ulceration capacity of the gastric lining when compared to salicylic acid - drug available in the treatment.

The classification of the compound at grade 1 is resulting from the observation of small microlesions (less than 1 mm) on the stomach lining.

### Treatment with the compound N-(1,3-dioxy-1 ,3-dihydro-2H-isoindol-2-yl)-2-(4-isobutylphenyl) propanamide. (Example 2.3)

We observed the stomach of the six animals treated with the new anti-inflammatory agent (example 2.3) derived from phthalimides and compared with ibuprofen (anti-inflammatory of reference in this test). Unlike ibuprofen, which showed lesions classified as grade 5, the compound of Example 2.3 showed no lesion on the gastric lining. This finding allowed classifying it as grade zero. There was also a little change in color of the mucosa of animals treated with the compound of Example 2.3.

However this small change in color does not affect the improved activity of the compound of Example 2.3 which seems to be safer than ibuprofen for chronic treatments where it is necessary to administer the daily dosage of the anti-inflammatory agent.

### Treatment with the compound N-(1,3-dioxy-1 ,3-dihydro-2H-isoindol-2-yl)-2-(6-methoxy-2-naphtyl) propanamide.

### (Example 3.3)

We observed the stomach of the six animals treated with the new anti-inflammatory agent (compound of Example 3.3) derived from phthalimides and compared with naproxen (anti-inflammatory of reference in this test). Unlike naproxen, which showed lesions classified as grade 5, the compound of Example 3.3 showed no lesion on the gastric lining. This finding allowed classifying it as grade zero.

We did not observe any - macro- and microscopically - morphological change or in the color of the stomach tissue, which proved to be similar to the stomach of the rats where saline was administered.

### Treatment with the compound 2 - {2 - [(2,6-dichlorophenyl) amine] phenyl}-N-(1,3-dioxy-1 ,3-dihydro-2H-isoindol-2-yl) acetamide (Example 5.3)

We observed the stomach of the six animals treated with the new anti-inflammatory agent (compound of Example 5.3) derived from phthalimides and compared with diclofenac (anti-inflammatory of reference in this test). Unlike diclofenac, which showed lesions classified as grade 5, the compound of Example 5.3 showed no lesion on the gastric lining. This finding allowed classifying it as grade zero. There was also a little change in the color of the mucosa of the animals treated with the compound of Example 5.3.

However this small change in color does not affect the improved activity of the compound of Example 5.3 which seems to be safer than diclofenac for chronic treatments where it is necessary to administer the daily dosage of the anti-inflammatory agent.

### Treatment with the compound 2 - {2 - [(2-chloro-6-fluorophenyl) amine]-5-methylphenyl}-N-(1,3-dioxy-1 ,3-dihydro-2H-isoindol-2-yl) acetamine (Example 6.3)

We observed the stomach of the six animals treated with the new anti-inflammatory agent (compound of Example 6.3) derived from phthalimides and compared with lumiracoxib (anti-inflammatory of reference in this test). Unlike lumiracoxib, which showed lesions classified as grade 2, the compound of Example 6.3 showed no lesion on the gastric lining. This finding allowed classifying it as grade zero.

We did not observe any - macro- and microscopically - morphological change or in the color of the stomach tissue, which proved to be similar to the stomach of the rats where saline was administered.

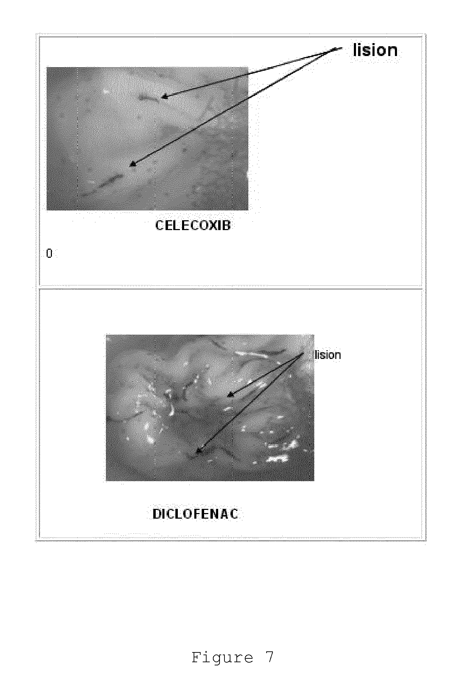

Through Figure 6 we can see that the compounds of the phthalimide derivatives did not cause gastric ulceration in the stomachs of rats treated at doses of 100 µmol/kg, which was the dose that showed anti-inflammatory activity. The gastric mucosa showed a similar appearance to saline, showing that the new compounds of the invention are safer with regard to gastroulceration, when compared with the anti-inflammatory agents available in therapy, such as diclofenac and celecoxib (see Figure 7).

Figure 7 shows photographs of the stomach of rats treated with diclofenac (grade of lesion = 5) or celecoxib (grade of lesion = 2) showing their ulcers caused by the administration of the anti-inflammatory drug in a single dose at the dose of 100 µmol/kg.

In Figure 7 we selected the stomach of the animals treated with diclofenac and celecoxib, respectively. Celecoxib was intended as a selective inhibitor of isoform COX-2 present in high concentrations of the inflammatory process. COX-2 converts arachidonic acid into prostaglandins, which among the various actions can cause sensitivity of the nociceptive terminals, vasodilatation and extravasation of inflammatory exudate, leading to edema and pain. Through Figure 7, it can be seen that even the COX-2 selective NSAID has the potential to cause gastric ulceration, which according to the classification reported in the literature can be classified as grade 2. The NSAIDs that are not selective, such as for instance, diclofenac, present maximum ulcerogenic potential and, thus, are classified as grade 5. Although the non-selective NSAIDs, in a general way, can be potentially ulcerogenic, the degree of ulceration varies from one anti-inflammatory agent to another. Thus, it is observed that the NSAID, such as for example, the salicylic acid (grade 5) is a lot more ulcerogenic than diclofenac (also grade 5). That which differs one from the other is the size of the lesions observed in different NSAIDs.

By evaluating the potential gastroulceration of the new anti-inflammatory agents of the invention, one can infer that when compared to NSAIDs available in the market, the compounds of this invention are safer and less ulcerogenic, justifying their use for patients who need the daily use of the anti-inflammatory agents.

### EXAMPLE 14

### Abdominal contortion test induced by acetic acid using the compounds of the invention obtained in examples 1.3, 3.3, 4.3 and 5.3

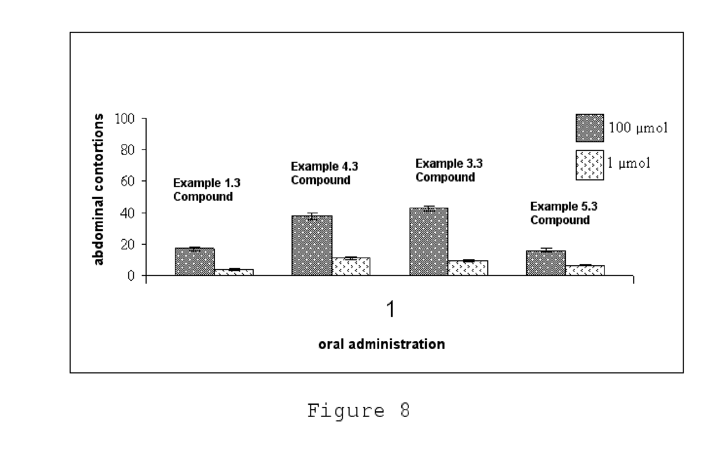

Additionally, we conducted the evaluation of possible adverse reactions of the compounds of the invention through the abdominal contortion test induced by acetic acid. The following compounds of the invention were used in the tests: N-(1,3-dioxy-1,3-dihydro-2H-isoindol-2-yl)-2-hydroxybenzamide (compound obtained in accordance with Example 1.3), N-(1,3-dioxy-1,3-dihydro-2H-isoindol-2-yl)-2-(6-methoxy-2-naphtyl)propanamide (compound obtained in accordance with Example 3.3), 2-(3-benzoylphenyl)-N-(1,3-dioxy-1,3-dihydro-2H-isoindol-2-yl)propanamide (compound obtained in accordance with Example 4.3), 2-{2-[(2,6-dichlorophenyl)amino]phenyl}-N-(1,3-dioxy-1,3-dihydro-2H-isoindol-2-yl)acetamide (compound obtained in accordance with Example 5.3). The results are represented in Figure 8. In the test, the control used was gum Arabic 5% which does not present analgesic activity because it was considered as being 0% of inhibition.

Figure 8 shows the percentage of inhibition of abdominal contortions induced by acetic acid 0.1 N in the presence of the hybrid compounds of the invention at concentrations of 1 µmol/kg and 100 µmol/kg. Gum Arabic 5%, as control, showed no analgesic activity.

From Figure 8 one can observe that the hybrid compound derived from the salicylic acid of the invention (compound of Example 1.3) presented at a concentration of 100 µmol/kg inhibitory activity of abdominal contortion of around 17.2%, demonstrating that this new derivative showed no analgesic activity.

On the other hand, the compounds of Examples 3.3, 4.3 and 5.3 presented at a concentration of 100 µmol/kg inhibitory activity of abdominal contortion of around 42.5%, 37.9% and 16.2%, respectively. From the chart, one can observe that the compounds of the invention presented peripheral analgesic activity being noteworthy to highlight the analgesic activities of naproxen and ketoprofen that inhibited the abdominal contortions in about 43 to 40%.

These results were tested at a concentration of 1 µmol/kg, from where one can see that all compounds of the invention tested showed peripheral analgesic activity. From Figure 8 it is noted that the response was dose-dependent.

The reports in the literature point to the fact that drugs, such as, for example, diclofenac, do not have analgesic activity seen in this model. Thus, the activity in 16.2% observed in diclofenac hybrid of this invention is due in part to the introduction of the phthalmidic subunit present in the molecule of thalidomide and held responsible for this analgesic effect.

From the results obtained, we can see that the new anti-inflammatory agents derived from phthalimides have peripheral analgesic activity in the model of abdominal contortion induced by acetic acid.

In Table 2 it is also possible to evaluate the analgesic activity of compounds of the invention that were tested at a concentration of 100 µmol/kg.

**Table 2: Percentage of inhibition of abdominal contortions obtained by use of the compounds of the invention**

| | | Compound | | | |
|---|---|---|---|---|---|
| | **control Gum Arabic** | **Example 1.3** | **Example 3.3** | **Example 4.3** | **Example 5.3** |
| | **5%** | | | | |
| **Average** | 65.83 | 54.5 | 37.8 | 40.8 | 55.1 |
| **% of inhibition** | 0 | 17.2 | 42.5 | 37.9 | 16.2 |
| **EMW** | 2.07 | 2.6 | 3.1 | 3.6 | 1.81 |

### Methodologies used for the pharmacological tests

Additionally, we report the methodologies used in the pharmacological tests described above, in which the compounds of the invention were used.

### Rat paws edema induced by carrageenan (see BANDARAGE et al 2000)

The methodology to be reported can be found in detail in Bandarage, U.K.; Chen, L.; Fang, X.; Garvey, D.S.; Glavin, A.; Janero, D.R.; Letts, L.G.; Mercer, G.J.; Saha, J.K.; Schroeder, J.D.; Shumway, M.J.; Tam, S.W. Nitrosothiol Esters of Diclofenac: Synthesis and Pharmacological Characterization of the Gastrointestinal-Sparing Prodrugs. J. Med. Chem. v.43, p. 4005-4016, 2000.

The injection of carrageenan in Wistar rat paw causes edema and local inflammation which can be seen by the increased thickness of the rat paw. Pre-treatment with compounds that have anti-inflammatory activity decreases the edema and inflammation caused by carrageenan administration.

The compounds of this invention have been administrated one (1) hour before inoculation of the irritating agent, carrageenan in the paws of the animals with the aid of a gavage tube orally, using water as a solvent.

The monitoring of the inflammation and the anti-inflammatory activity of the compounds of the invention was made by measuring the thickness in millimeters, of the rat's paw.

The control group received sub plantar injection, in the hind paws, the irritating chemical agent, carrageenan, and saline solution, orally.

The hind paws were measured before treatment and hourly for 6 hours after administration of carrageenan, through the thickness to determine their volumes (in mm). The results were expressed as the difference between the readings of the paws before and after treatments.

### Gastric Lesion Test (see BANDARAGE et al 2000)

In order to characterize the profile of gastric lesion caused by the new anti-inflammatory derivatives of the invention the extent of the lesions caused by the administration of said anti-inflammatory agents was measured.

The gastric ulcerogenesis was verified in the same animals from the groups used for the paw's edema model.

After six hours of readings of the legs, the animals were euthanized in CO₂ and had their stomachs removed, opened towards greater curvature and washed with saline.

Through the exposure of the mucosa its color and integrity were shown. In the case of the existence of lesions, these were counted and measured according to the rate of gastric ulcerogenesis (IUG), which abides by numerical criteria for the classification of gastric mucosal lesions: (lesions < 1 mm = 1 ; 1.5 to 2.5 mm = 2 ; 2.5 to 3.5 mm = 3, 3.5 to 4.5 mm = 4 and > 4.5 mm = 5). The results obtained were reported as averages ± E. P. M.

### Abdominal contortion Test Induced by Acetic Acid (see Collier et al 1968)

The methodology to be reported can be found in detail in (COLLIER, H. O. J.; DINNEEN, L. C.; JOHNSON, C.A.; SCHNEIDER, C. Abdominal constriction response and its suppression by analgesic drugs in the mouse. British Journal of Pharmacology and Chemotherapy. v.32, n.2, p.295-310, 1968).

The antinociceptive profile was evaluated by testing abdominal contortions induced by acetic acid in mice. In this assay, there were used Swiss mice of both sexes weighing between 21 and 28 g, fasted for a period of approximately 8 hours. The test-substance was administered orally and had as the gum Arabic at 5% as vehicle. One hour later, it was administered acetic acid at 0.1 N (0.1 mL/10g weight) in the peritoneal cavity of the animals. Ten minutes after injection of acetic acid, the contortions were counted for 20 minutes. Controls were made of the vehicle (gum Arabic) and these showed no pharmacological activity.

### Statistical Analysis

Grades of significance between the experimental and control groups were made using the Student's t test. Values were considered significant when *P < 0.05. The results were expressed as average ± standard average error, as indicated in the captions of the figures.

All publications and patent applications mentioned in the description are indicative of the grade of those specialists in the technique to which the invention relates. All publications and patent applications are incorporated herein for reference to the same extent as if each individual publication or each patent application had been specifically and individually indicated to be incorporated for reference.

Although the foregoing invention has been described in some detail by way of illustration and examples for purposes of clarity and understanding, it will be obvious that certain changes and modifications may be practiced within the scope of the claims accompanying this description.

## Claims

1. Phthalimide derivative Compound from non-steroidal anti-inflammatory Compounds comprising the Formula (I): 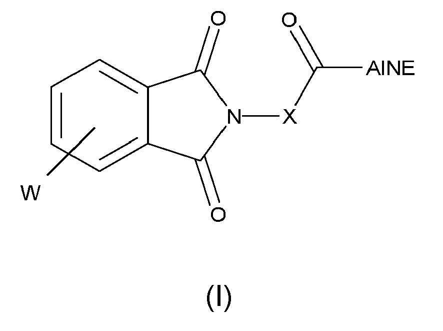 Where:
AINE (NSAID) represents a non-steroidal anti-inflammatory **Compound** containing at least one carboxylic acid subunit;
W represents one or more independently selected substituents from the group consisting of F, Cl, Br, NO₂, NH₂, OH, OCH₃, OCF₃, CF₃, CH₃;
X is selected from the group consisting of: (CH₂)ₙ O-, 2-hydroxy-phenyl, 3-hydroxy-phenyl, 4-hydroxy-phenyl, 2-hydroxy-benzyl, 3-hydroxy-benzyl, 4-hydroxy-benzyl, 2-hydroxy-ethylbenzyl, 3 - ethylbenzyl-hydroxy, 4-hydroxy-benzyl ethylbenzyl, thiophene, furan, pyrrole, 2-hydroxy-pyridine, 3-hydroxy-pyridine, 4-hydroxy-pyridine, pyrazine, pyrimidine, benzothiophene, benzofurans, indole, quinoline, isoquinoline, naphthalene, CH₂-2-hydroxy-thiophene, CH₂-3-hydroxy-thiophene, CH₂-2-hydroxy-furan, CH₂-3-hydroxy-furan, CH₂CH₂-2-hydroxy-thiophene, CH₂CH₂ -3-hydroxy-thiophene, CH₂CH₂-2-hydroxy-furan, CH₂CH₂-3-hydroxy-furan, 2-amine-phenyl, 3-amine-phenyl, 4-amine-phenyl, 2-amine-benzyl, 3-amine-benzyl, 4-amine-benzyl, 2-amine-ethylbenzyl, 3-amine-ethylbenzyl, 4-amine-ethylbenzyl benzyl, thiophene, furan, pyrrole, 2 -amine-pyridine, 3-amine-pyridine, 4-amine-pyridine, pyrazine, pyrimidine, benzotiophen, benzofurans, indole, quinoline, isoquinoline, naphthalene, CH₂-2-amine-thiophene, CH₂-3-amine-thiophene, CH₂ -2-amine-furan, CH₂-3-amine-furan, CH₂CH₂-2-amine-thiophene, CH₂CH₂-3-amine-thiophene, CH₂CH₂-2-amine-furan, CH₂CH₂-3-amine-furan, and
n is selected from 0, 1, 2, 3, 4, 5, 6.

2. **Compound** according to claim 1 wherein the NSAID component is selected from the group consisting of diclofenac, lumiracoxib, naprofen, ibuprofen, salicylic acid, mesalamine, olsalazine, indomethacin, aceclofenac and ketoprofen.

3. **Compound** according to claim 1 wherein said **Compound** is selected from the group consisting of:
- 2-(2-(2,6-dichlorophenylamine) phenyl)-N-(1,3-dioxyisoindolin-2-yl) acetamide - **Compound** 1
- 2-(1,3-dioxyisoindolin-2-yl) methyl-(2-(2,6-dichlorophenyl amine) phenyl) acetate - **Compound** 2
- 2-(1,3-dioxyisoindolin-2-yl) ethyl-(2-(2-(2,6-dichlorophenyl amine) phenyl) acetate - **Compound** 3
- (4-(1,3-dioxyisoindolin-2-yl) phenyl) ethyl 2-(2-(2,6-dichlorophenylamine) phenyl) acetate - **Compound** 4
- 4-(1,3-dioxyisoindolin-2-yl) benzyl 2-(2-(2,6-dichlorophenyl amine) phenyl) acetate - **Compound** 5
- 3-(1,3-dioxyisoindolin-2-yl) benzyl 2-2-(2-(2,6-dichlorophenyl amine) phenyl) acetate - **Compound** 6
- 2-(1,3-dioxyisoindolin-2-yl) benzyl 2-(2-(2-(2,6-dichlorophenyl amine) phenyl) acetate - **Compound** 7
- 2-(2-(2-chloro-6-fluorophenylamine)-5-methyl-phenyl)-N-(1,3-dioxyisoindolin-2-yl) acetamide - **Compound** 8
- (1,3-dioxyisoindolin-2-yl) methyl 2-(2-(2-chloro-6-fluoro phenylamine)-5 methylphenyl) acetate - **Compound** 9
- (1,3-dioxyisoindolin-2-yl) ethyl 2-(2-(2-chloro-6-fluorophenyl amine)-5 methylphenyl) acetate - **Compound** 10
- 2-(4-(1,3-dioxyisoindolin-2-yl) phenyl) ethyl 2-(2-(2-chloro-6-fluoro phenylamine)-5-methylphenyl) acetate - **Compound** 11
- (4-(1,3-(dioxyisoindolin-2-yl) phenyl) methyl 2-(2-(2-chloro-6-fluoro phenylamine)-5-methylphenyl) acetate - **Compound** 12
- (3-(1,3-(dioxyisoindolin-2-yl) phenyl) methyl 2-(2-(2-chloro-6-fluoro phenylamine)-5-methylphenyl) acetate - **Compound** 13
- (2-(1,3-(dioxyisoindolin-2-yl) phenyl) methyl 2-(2-(2-chloro-6-fluoro phenylamine)-5-methylphenyl) acetate - **Compound** 14
- 2-(2-methoxynaphthalene-6-yl)-N-(1,3-dioxyisoindolin-2-yl) propanamide - **Compound** 15
- (1,3-dioxyisoindolin-2-yl) methyl 2-(2-methoxynaphthalene-6-yl) propanoate - **Compound** 16
- (1,3-dioxyisoindolin-2-yl) ethyl 2-(2-methoxynaphthalene-6-yl) propanoate - **Compound** 17
- 2-(4-(1,3-dioxyisoindolin-2-yl) phenyl) ethyl 2-(2-methoxy naphthalene-6-yl) propanoate - **Compound** 18
- 2-(4-(1,3-dioxyisoindolin-2-yl) phenyl) ethyl 2-(2-methoxy naphthalene-6-yl) propanoate - **Compound** 19
- (3-(1,3-dioxyisoindolin-2-yl) phenyl) methyl 2-(2-methoxy naphthalene-6-yl) propanoate - **Compound** 20
- (2-(1,3-dioxyisoindolin-2-yl) phenyl) methyl 2-(2-methoxy naphthalene-6-yl) propanoate - **Compound** 21
- 2-hydroxy-N-(1,3-dioxyisoindolin-2-yl) benzamide - **Compound** 22
- 2-(1,3-dioxyisoindolin-2-yl) methyl 2-hydroxybenzoate - **Compound** 23
- 2-(1,3-dioxyisoindolin-2-yl) ethyl 2-hydroxybenzoate - **Compound** 24
- 2-(4-(1,3-dioxyisoindolin-2-yl) phenyl) ethyl 2-hydroxy benzoate - **Compound** 25
- 4-(1,3-dioxyisoindolin-2-yl) phenyl) methyl 2-hydroxy benzoate - **Compound** 26
- (3-(1,3-dioxyisoindolin-2-yl) phenyl) methyl 2-hydroxybenzoate - **Compound** 27
- (2-(1,3-dioxyisoindolin-2-yl) phenyl) methyl 2-hydroxybenzoate - **Compound** 28
- 2-(4-isobutylphenyl)-N-(1,3-dioxyisoindolin-2-yl) propanamide - **Compound** 29
- 1,3-dioxyisoindolin-2-yl) methyl 2-(4-isobutylphenyl) propanoate - **Compound** 30
- 2-(1,3-dioxyisoindolin-2-yl) ethyl 2-(4-isobutylphenyl) propanoate - **Compound** 31
- 2-(4-(1,3-dioxyisoindolin-2-yl) phenyl) ethyl 2-(4-isobutyl phenyl) propanoate - **Compound** 32
- (4-(1,3-dioxyisoindolin-2-yl) phenyl) methyl 2 (4-isobutyl phenyl) propanoate - **Compound** 33
- (3-(1,3-dioxyisoindolin-2-yl) phenyl) methyl 2(4-isobutyl phenyl)propanoate - **Compound** 34
- 2-(1,3-dioxyisoindolin-2-yl) phenyl) methyl 2(4-isobutyl phenyl) propanoate - 35
- 2-(3-benzoylphenyl)-*N*-(1,3-dioxy-1,3-dihydro-2*H*-isoindol-2-yl) propanamide - **Compound** 36
- 1,3-dioxy-1,3-dihydro-2*H*-isoindol-2-yl) methyl 2-(3-benzoylphenyl) propanoate - **Compound** 37
- (1,3-dioxy-1,3-dihydro-2*H*-isoindol-2-yl) ethyl 2-(3-benzoylphenyl) propanoate - **Compound** 38
- 4-(1,3-dioxyisoindolin-2-yl) phenyl) ethyl 2-(3-benzoylphenyl) propanoate - **Compound** 39
- 4-(1,3-dioxyisoindolin-2-yl) phenyl) methyl 2-(3-benzoylphenyl) propanoate - **Compound** 40
- 3-(1,3-dioxyisoindolin-2-yl) phenyl) methyl 2-(3-benzoylphenyl) propanoate - **Compound** 41
- 2-(1,3-dioxyisoindolin-2-yl) phenyl) methyl 2-(3-benzoylphenyl) propanoate - **Compound** 42
- 2-[1-(4-clorobenzoyl)-5-metoxy-2-methyl -1*H*-indol-3-yl]acetohydrazidr- N-(1,3-dioxyisoindolin-2-yl) - **Compound** 43
- (1,3-dioxy-1,3-dihydro-2*H*-isoindol-2-yl) methyl [1-(4-chlorobenzoyl)-5-metoxy-2-methyl -1*H*-indol-3-yl] acetate - **Compound** 44
- (1,3-dioxy-1,3-dihydro-2*H*-isoindol-2-yl) ethyl [1-(4-chlorobenzoyl)-5-methoxy-2-methyl -1*H*-indol-3-yl]acetate - **Compound** 45
- (4-(1,3-dioxyisoindolin-2-yl) phenylethyl [1-(4-chlorobenzoyl)-5-methoxy-2-methyl -1*H*-indol-3-yl]acetate-**Compound** 46
- (4-(1,3-dioxyisoindolin-2-yl) phenylmethyl [1-(4-chlorobenzoyl)-5-methoxy-2-methyl -1*H*-indol-3-yl]acetate - **Compound** 47
- (3-(1,3-dioxyisoindolin-2-yl)phenylmethyl [1-(4-chlorobenzoyl)-5-methoxy-2-methyl -1*H*-indol-3-yl]acetate - **Compound** 48
- (2-(1,3-dioxyisoindolin-2-yl)phenylmethyl [1-(4-clorobenzoyl)-5-methoxy-2-methyl -1*H*-indol-3-yl]acetate - **Compound** 49
- 3,3'-(*E*)-diazene-bis-2-hydroxy-N-(1,3-dioxyisoindolin-2-yl)benzamide- **Compound** 50
- bis-(1,3-dioxy-1,3-dihydro-2*H*-isoindol-2-yl)methyl -3,3'-(E)-diazene-1,2-diylbis(6-hydroxybenzoate) - **Compound** 51
- bis-(1,3-dioxy-1,3-dihydro-2*H*-isoindol-2-yl)ethyl -3,3'-(E)-diazene-1,2-diylbis(6-hydroxybenzoate) - **Compound** 52
- bis-4-(1,3-dioxyisoindolin-2-yl)phenyl)ethyl -3,3'-(*E*)-diazene-1,2-diylbis(6-hydroxybenzoate)- **Compound** 53
- bis-4-(1,3-dioxyisoindolin-2-yl)phenyl)methyl -3,3'-(E)-diazene-1,2-diylbis(6-hydroxybenzoate) - **Compound** 54
- bis-3-(1,3-dioxyisoindolin-2-yl)phenyl)methyl -3,3'-(E)-diazene-1,2-diylbis(6-hydroxybenzoate) - **Compound** 55
- bis-2-(1,3-dioxyisoindolin-2-yl)phenyl)methyl -3,3'-(E)-diazene-1,2-diylbis(6-hydroxybenzoate) - **Compound** 56
- 4-{(*E*)-[3-(hidrazinocarbonyl-N-(1,3-dioxyisoindolin-2-yl))-4-hydroxyphenyl]diazenyl}-N-pyridin-2-yl benzenesulfonamide - **Compound** 57
- (1,3-dioxy-1,3-dihydro-2*H*-isoindol-2-yl)methyl 2-hydroxy-5-[(E)-{4-[(pyridin-2-ylamine)sulfonyl]phenyl}diazenyl]benzoate - **Compound** 58
- (1,3-dioxy-1,3-dihydro-2*H*-isoindol-2-yl)ethyl 2-hydroxy-5-[(*E*)-{4-[(pyridin-2-ylamine)sulfonyl]phenyl} diazenyl]benzoate - **Compound** 59
- (4-(1,3-dioxyisoindolin-2-yl)phenyl)ethyl 2-hydroxy-5-[(*E*)-{4-[(pyridin-2-ylamine)sulfonyl]phenyl}diazenyl]benzoate - **Compound** 60
- 4-(1,3-dioxyisoindolin-2-yl)phenyl)methyl 2-hydroxy-5-[(*E*)-{4-[(pyridin-2-ylamine)sulfonyl]phenyl}diazenyl]benzoate - **Compound** 61
- 3-(1,3-dioxyisoindolin-2-yl)phenyl)methyl 2-hydroxy-5-[(*E*)-{4-[(pyridin-2-ylamine)sulfonyl]phenyl}diazenyl]benzoate - **Compound** 62
- 2-(1,3-dioxyisoindolin-2-yl)phenyl)methyl 2-hydroxy-5-[(*E*)-{4-[(pyridin-2-ylamine)sulfonyl]phenyl}diazenyl]benzoate - **Compound** 63.

4. **Compound** in accordance with claim 3 **characterized by** the fact that said **compound** é selected from the group consisting of : acid N-(1,3-dioxy-1,3-dihydro-2H-isoindol-2-yl)-2-hydroxybenzamide; N-(1,3-dioxy-1,3-dihydro-2H-isoindol-2-yl)-2-(4-isobutylphenyl)propanamide; N-(1,3-dioxy-1,3-dihydro-2H-isoindol-2-yl)-2-(6-methoxy-2-naphtyl) propanamide; 2-(3-benzoylphenyl)-N-(1,3-dioxy-1,3-dihydro-2H-isoindol-2-yl)propanamide; 2-{2-[(2,6-dichlorophenyl)amine] phenyl}-N-(1,3-dioxy-1,3-dihydro-2H-isoindol-2-yl)acetamide; 2-{2-[(2-chloro-6-fluorophenyl)amine]-5-methyl phenyl}-N-(1,3-dioxy-1,3-diiidro-2H-isoindol-2-yl)acetamide; 2-(1,3-dioxy-1,3-dihydro-2H-isoindol-2-yl)ethyl -{2-[(2,6-dichlorophenyl) amine]phenyl} acetate and (1,3-dioxy-1,3-dihydro-2*H-*isoindol-2-yl)methyl {2-[(2,6-dichlorophenyl)amine]phenyl}acetate.

5. Process to obtain phthalimide derivatives of non-steroidal anti-inflammatory functioning **Compounds** having the general Formula (I) 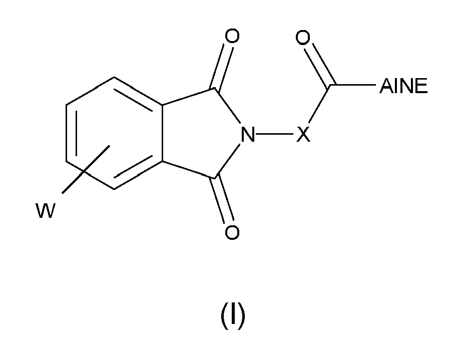 wherein it consists of the stages of: (i) esterification reaction of NSAID by acid catalysis exploring nucleophilicity of alcohols of low molecular weight; (ii) nucleophilic substitution by hydrazine or condensation of duly functioning with anhydrites in an appropriate solvent and (iii) condensation of hydrazides duly functionalized with a reagent selected from the anhydride group into a proper solvent and (iv) separation of the functioning phthalimide derivative desired from the **Compounds** estimated in the general Formula (I).

6. Process in accordance with claim 5 wherein in stage (i) the alcohol is methanol, ethanol or propanol.

7. Process in accordance with claim 5 wherein in stage (ii) the obtainment of hydrazide is performed by nucleophilic substitution of the ester using hydrazine hydrate 25%-98%.

8. Process in accordance with claim 5 wherein in stage (ii) the obtainment of hydrazide is performed by coupling reaction between the carboxylic acid of NSAID and hydrazine hydrate using coupling agents.

9. Process in accordance with claim 5 wherein in stage (iii) the condensation of duly functioning is performed with anhydrides, preferably phthalic anhydride.

10. Process to obtain phthalimide derivatives from functioning non-steroidal anti-inflammatory **Compounds** having the general Formula (I) 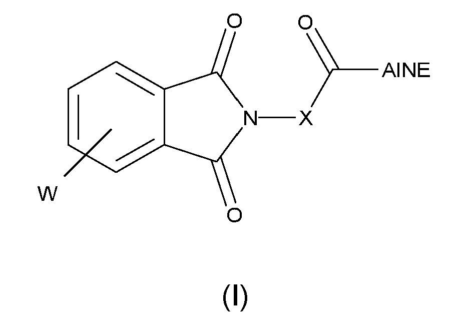 wherein it consists of the stages of: (i) preparation of duly functioning phthalimide derivatives, (ii) activation reaction of the carboxylic acid the NSAID with coupling agent (iii) condensation of the duly functioning phthalimide derivate with the NSAID carboxylic acid group of the previously activated in an appropriate solvent and (iv) separation of the desired functioning phthalimide derivate from the **Compounds** foreseen in the general Formula (I)

11. Process in accordance with claim 10 wherein in stage (i) the functioning phthalimide derivative is obtained through the condensation reaction of functionalized amines with phthalic anhydride, phthalic acid or halides of phthalic acid in appropriate solvents such as diethyl ether, tetrahydrofuran, dichloromethane, chloroform, glacial acetic acid, pyridine or toluene;

12. Process in accordance with claim 10 wherein said desired functioning phthalic derivate is one of the **Compounds** as defined in claim 1.

13. Process in accordance with claim 10 wherein in stage (ii) the activating agent is **characterized by** dicloexylcarbodiimide (DCC).

14. Process in accordance with claim 10 wherein in stage (iii) the solvent is dichloromethane, chloroform or pyridine.

15. Process to obtain phthalimide derivatives from functioning non-steroidal anti-inflammatory **Compounds** having the general Formula (I) 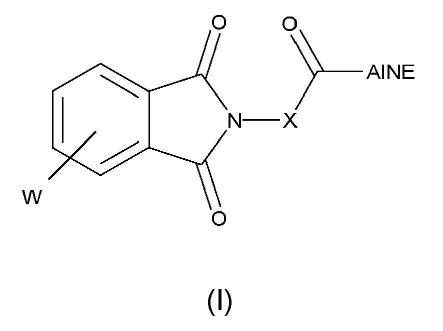 wherein it consists of the stages of: (i) the halogenation reaction of the carboxylic acid of NSAID with halogenating agent suitable for obtaining the corresponding acyl halide (ii) preparation of phthalimide derivative properly functioning, (iii) condensation of the phthalimide derivate containing alcohol or free amine function with the obtained acyl halide; (iv) separation of the desired functioning phthalimide derivative among the **Compounds** foreseen in the general Formula (I)

16. Process in accordance with claim 15 wherein in stage (i) the appropriate halogenating agent is selected from the group consisting of oxalyl chloride and thionyl chloride.

17. Process in accordance with claim 15 wherein in stage (ii) the functioning phthalmide derivative is obtained by the condensation reaction of amines functioning with phthalic anhydride.

18. Process in accordance with claim 15 wherein said desired functioning phthalmide derivative is one of the **Compounds** as defined in claim 1.

19. Process in accordance with claim 15 wherein in stage (iii) the condensation reaction is carried out in solvent such as dichloromethane, chloroform, toluene, tetrahydrofuran, dimethyl formamide or pyridine.

20. Process according to claims 10 and 15 wherein they are obtained from prodrugs containing ester or amide functional group.

21. Pharmaceutical composition comprising:
(a) a therapeutically effective amount of at least one phthalmide non-steroidal anti-inflammatory derivative functioning **Compound** as defined in claim 1;
(b) optionally, a pharmacologically effective amount of at least one active ingredient suitable for the treatment of a medical condition involving pain or an inflammatory disorder, and
(c) a pharmaceutically acceptable vehicle.

22. Composition according to claims 10 and 15 wherein at least said phthalmide derivative of functioning non-steroidal anti-inflammatory **Compounds** is selected from the group consisting of:
- 2-(2-(2,6-dichlorophenylamine) phenyl)-N-(1,3-dioxyisoindolin-2-yl) acetamide - **Compound** 1
- 2-(1,3-dioxyoisoindolin-2-yl) methyl 2 - (2 - (2,6-dichlorophenyl amine) phenyl) acetate - **Compound** 2
- 2-(1,3-dioxyisoindolin-2-yl) ethyl-(2-(2-(2,6-dichlorophenyl amine) phenyl) acetate - **Compound** 3
- (4-(1,3-dioxyisoindolin-2-yl) phenyl) ethyl 2 -(2-(2,6-dichlorophenylamine) phenyl) acetate - **Compound** 4
- 4-(1,3-dioxyisoindolin-2-yl)benzyl 2-(2-(2,6-dichlorophenyl amine)phenyl) acetate - **Compound** 5
- 3-(1,3-dioxyisoindolin-2-yl)benzyl 2-(2-(2,6-dichlorophenyl amine)phenyl) acetate - **Compound** 6
- 2-(1,3-dioxyisoindolin-2-yl)benzyl 2-(2-(2,6-dichlorophenyl amine)phenyl) acetate - **Compound** 7
- 2-(2-(2-chloro-6-fluorophenylamine)-5-methyl -phenyl)-N-(1,3-dioxyisoindolin-2-yl)acetamide - **Compound** 8
- (1,3-dioxyisoindolin-2-yl)methyl 2-(2-(2-chloro-6-fluoro phenylamine)-5-methyl phenyl)acetate - **Compound** 9
- (1,3-dioxyisoindolin-2-yl)ethyl 2-(2-(2-chloro-6-fluorophenyl amine)-5-methyl phenyl)acetate - **Compound** 10
- 2-(4-(1,3-dioxyisoindolin-2-yl)phenyl)ethyl 2-(2-(2-chloro-6-fluorophenylamine)-5-methyl phenyl)acetate - **Compound** 11
- (4-(1,3-(dioxyisoindolin-2-yl)phenyl)methyl 2-(2-(2-chloro-6-fluorophenylamine)-5-methyl phenyl)acetate - **Compound** 12
- (3-(1,3-(dioxyisoindolin-2-yl)phenyl)methyl 2-(2-(2-chloro-6-fluorophenylamine)-5-methyl phenyl)acetate - **Compound** 13
- (2-(1,3-(dioxyisoindolin-2-yl)phenyl)methyl 2-(2-(2-chloro-6-fluorophenylamine)-5-methyl phenyl)acetate - **Compound** 14
- 2-(2-methoxynaphthalene-6-yl)-N-(1,3-dioxyisoindolin-2-yl) propanamide - **Compound** 15
- (1,3-dioxyisoindolin-2-yl) methyl 2-(2-methoxynaphthalene-6-yl)propanoate - **Compound** 16
- (1,3-dioxyisoindolin-2-yl) ethyl 2-(2-methoxynaphthalene-6-yl) propanoate - **Compound** 17
- 2-(4-(1,3-dioxyisoindolin-2-yl)phenyl) ethyl 2-(2-methoxynaphthalene-6-yl)propanoate - **Compound** 18
- 2-(4-(1,3-dioxyisoindolin-2-yl)phenyl) ethyl 2-(2-methoxynaphthalene-6-yl)propanoate - **Compound** 19
- (3-(1,3-dioxyisoindolin-2-yl)phenyl) methyl 2-(2-methoxynaphthalene-6-yl) propanoate - **Compound** 20
- (2-(1,3-dioxyisoindolin-2-yl)phenyl) methyl 2-(2-methoxynaphthalene-6-yl) propanoate - **Compound** 21
- 2-hydroxy-N-(1,3-dioxyisoindolin-2-yl) benzamide - Compound 22
- 2-(1,3-dioxyisoindolin-2-yl)methyl 2-hydroxybenzoate - **Compound** 23
- 2-(1,3-dioxyisoindolin-2-yl)ethyl 2-hydroxybenzoate - **Compound** 24
- 2-(4-(1,3-dioxyisoindolin-2-yl)phenyl) ethyl 2-hydroxybenzoate - **Compound** 25
- 4-(1,3-dioxyisoindolin-2-yl)phenyl) methyl 2-hydroxybenzoate - **Compound** 26
- (3-(1,3-dioxyisoindolin-2-yl)phenyl) methyl 2-hydroxybenzoate - **Compound** 27
- (2-(1,3-dioxyisoindolin-2-yl)phenyl) methyl 2-hydroxybenzoate - **Compound** 28
- 2-(4-isobutylphenyl)-N-(1,3-dioxyisoindolin-2-yl) propanamide - **Compound** 29
- 1,3-dioxyisoindolin-2-yl)methyl 2-(4-isobutylphenyl) propanoate - **Compound** 30
- 2-(1,3-dioxyisoindolin-2-yl)ethyl 2-(4-isobutylphenyl) propanoate - **Compound** 31
- 2-(4-(1,3-dioxyisoindolin-2-yl)phenyl)ethyl 2-(4-isobutyl phenyl) propanoate - **Compound** 32
- (4-(1,3-dioxyisoindolin-2-yl)phenyl)methyl 2(4-isobutyl phenyl) propanoate - **Compound** 33
- (3-(1,3-dioxyisoindolin-2-yl)phenyl)methyl 2(4-isobutyl phenyl) propanoate - **Compound** 34
- 2-(1,3-dioxyisoindolin-2-yl)phenyl)methyl 2(4-isobutyl phenyl) propanoate - **Compound** 35
- 2-(3-benzoylphenyl)-*N*-(1,3-dioxy-1,3-dihydro-2*H*-isoindol-2-yl) propanamide - **Compound** 36
- 1,3-dioxy-1,3-dihydro-2*H*-isoindol-2-yl)methyl 2-(3-benzoylphenyl) propanoate - **Compound** 37
- (1,3-dioxy-1,3-dihydro-2*H*-isoindol-2-yl)ethyl 2-(3-benzoylphenyl) propanoate - **Compound** 38
- 4-(1,3-dioxyisoindolin-2-yl)phenyl) ethyl 2-(3-benzoylphenyl)propanoate - **Compound** 39
- 4-(1,3-dioxyisoindolin-2-yl)phenyl) methyl 2-(3-benzoylphenyl)propanoate - **Compound** 40
- 3-(1,3-dioxyisoindolin-2-yl)phenyl) methyl 2-(3-benzoylphenyl)propanoate - **Compound** 41
- 2-(1,3-dioxyisoindolin-2-yl)phenyl) methyl 2-(3-benzoylphenyl)propanoate - **Compound** 42
- 2-[1-(4-chlorobenzoyl)-5-methoxy-2-methyl -1*H*-indol-3-yl]acetohydrazide- N-(1,3-dioxyisoindolin-2-yl) - **Compound** 43
- (1,3-dioxy-1,3-dihydro-2H-isoindol-2-yl)methyl [1-(4-chlorobenzoyl)-5-methoxy-2-methyl -1*H*-indol-3-yl]acetate - **Compound** 44
- (1,3-dioxy-1,3-dihydro-2H-isoindol-2-yl) ethyl [1-(4-chlorobenzoyl)-5-methoxy-2-methyl -1H-indol-3-yl]acetate - **Compound** 45
- (4-(1,3-dioxyisoindolin-2-yl)phenyl)ethyl [1-(4-chloro benzoyl)-5-methoxy-2-methyl -1H-indol-3-yl]acetate - **Compound** 46
- (4-(1,3-dioxyisoindolin-2-yl)phenyl)methyl [1-(4-chloro benzoyl)-5-methoxy-2-methyl -1H-indol-3-yl]acetate - **Compound** 47
- (3-(1,3-dioxyisoindolin-2-yl)phenyl)methyl [1-(4-chloro benzoyl)-5-methoxy-2-methyl -1H-indol-3-yl]acetate - **Compound** 48
- (2-(1,3-dioxyisoindolin-2-yl)phenyl)methyl [1-(4-chloro benzoyl)-5-methoxy-2-methyl -1H-indol-3-yl]acetate - **Compound** 49
- 3,3'-(E)-diazene-bis-2-hydroxy-N-(1,3-dioxyisoindolin-2-yl)benzamidr- **Compound** 50
- bis-(1,3-dioxy-1,3-dihydro-2H-isoindol-2-yl)methyl -3,3'-(E)-diazene-1,2-diylbis(6-hydroxybenzoate) - **Compound** 51
- bis-(1,3-dioxy-1,3-dihydro-2*H*-isoindol-2-yl)ethyl -3,3'-(E)-diazene-1,2-diylbis(6-hydroxybenzoate) - **Compound** 52
- bis-4-(1,3-dioxyisoindolin-2-yl)phenyl)ethyl -3,3'-(*E*)-diazene-1,2-diylbis(6-hydroxybenzoate)- **Compound** 53
- bis-4-(1,3-dioxyisoindolin-2-yl)phenyl)methyl -3,3'-(E)-diazene-1,2-diylbis(6-hydroxybenzoate) - **Compound** 54
- bis-3-(1,3-dioxyisoindolin-2-yl)phenyl)methyl -3,3'-(E)-diazene-1,2-diylbis(6-hydroxybenzoate) - **Compound** 55
- bis-2-(1,3-dioxyisoindolin-2-yl)phenyl)methyl -3,3'-(E)-diazene-1,2-diylbis(6-hydroxybenzoate) - **Compound** 56
- 4-{(*E*)-[3-(hidrazinocarbonyl-N-(1,3-dioxyisoindolin-2-yl))-4-hydroxyphenyl]diazenyl}-*N*-pyridin-2-yl-benzeno sulfonamide - **Compound** 57
- (1,3-dioxy-1,3-dihydro-2*H*-isoindol-2-yl)methyl 2-hydroxy-5-[(*E*)-{4-[(pyridin-2-ylamine)sulfonyl]phenyl}diazenyl] benzoate - **Compound** 58
- (1,3-dioxy-1,3-dihydro-2*H*-isoindol-2-yl)ethyl 2-hydroxy-5-[(*E*)-{4-[(pyridin-2-ylamine)sulfonyl]phenyl}diazenyl] benzoate - **Compound** 59
- (4-(1,3-dioxyisoindolin-2-yl)phenyl)ethyl 2-hydroxy-5-[(*E*)-{4-[(pyridin-2-ylamine)sulfonyl]phenyl}diazenyl]benzoate - **Compound** 60
- 4-(1,3-dioxyisoindolin-2-yl)phenyl)methyl 2-hydroxy-5-[(*E*)-{4-[(pyridin-2-ylamine)sulfonyl]phenyl}diazenyl]benzoate - **Compound** 61
- 3-(1,3-dioxyisoindolin-2-yl)phenyl)methyl 2-hydroxy-5-[(*E*)-{4-[(pyridin-2-ylamine)sulfonyl]phenyl}diazenyl]benzoate - **Compound** 62
- 2-(1,3-dioxyisoindolin-2-yl)phenyl)methyl 2-hydroxy-5-[(*E*)-{4-[(pyridin-2-ylamine)sulfonyl]phenyl}diazenyl]benzoate - **Compound** 63.

23. Pharmaceutical composition according to claims 10 and 15 wherein at least said phthalmide derivative of functioning non-steroidal anti-inflammatory **Compounds** is selected from the group consisting of: acid N-(1,3-dioxy-1,3-dihydro-2H-isoindol-2-yl)-2-hydroxy benzamide; N-(1,3-dioxy-1,3-dihydro-2H-isoindol-2-yl)-2-(4-isobutylphenyl) propanamide; N-(1,3-dioxy-1,3-dihydro-2H-isoindol-2-yl)-2-(6-methoxy-2-naphtyl) propanamide; 2-(3-benzoylphenyl)-N-(1,3-dioxy-1,3-dihydro-2H-isoindol-2-yl) propanamide; 2-{2-[(2,6-dichlorophenyl)amine] phenyl}-N-(1,3-dioxy-1,3-dihydro-2H-isoindol-2-yl)acetamide; 2-{2-[(2-chloro -6-fluorophenyl)amine]-5-methyl phenyl}-N-(1,3-dioxy-1,3-dihydro-2H-isoindol-2-yl)acetamide; 2-(1,3-dioxy-1,3-dihydro -2H-isoindol-2-yl)ethyl -{2-[(2,6-dichlorophenyl) amine]phenyl} acetate and(1,3-dioxy-1,3-dihydro-2*H-*isoindol-2-yl)methyl {2-[(2,6-dichlorophenyl)amine]phenyl}acetate.

24. Use of a phthalimide **Compound** derived from non-steroidal anti-inflammatory functioning **Compounds** as defined in claims 1 to 4, wherein it is under medical conditions involving pain and inflammation.

25. Use in accordance with claim 24, wherein said phthalimide derived from non-steroidal anti-inflammatory functioning **Compounds** is selected from the group consisting of acid N-(1,3-dioxy-1,3-dihydro-2H-isoindol-2-yl)-2-hydroxybenzamide; N-(1,3-dioxy-1,3-dihydro-2H-isoindol-2-yl)-2-(4-isobutylphenyl)propanamide; N-(1,3-dioxy-1,3-dihydro-2H-isoindol-2-yl)-2-(6-metoxi-2-naftyl) propanamide; 2-(3-benzoylphenyl)-N-(1,3-dioxy-1,3-dihydro-2H-isoindol-2-yl)propanamide; 2-{2-[(2,6-diclorophenyl)amine] phenyl}-N-(1,3-dioxy-1,3-dihydro-2H-isoindol-2-yl)acetamide; 2-{2-[(2-cloro-6-fluorophenyl)amine]-5-methyl phenyl}-N-(1,3-dioxy-1,3-diiidro-2H-isoindol-2-yl)acetamide; 2-(1,3-dioxy-1,3-dihydro-2H-isoindol-2-yl)ethyl -{2-[(2,6-diclorophenyl) amine]phenyl} acetate e (1,3-dioxy-1,3-dihydro-2*H*-isoindol-2-yl)methyl {2-[(2,6-diclorophenyl)amine]phenyl} acetate.
